# EUROPEAN PATENT SPECIFICATION

(11) **EP 4 327 327 B1**
(45) Date of publication and mention of the grant of the patent: **01.10.2025**
(21) Application number: 22722627.1
(22) Date of filing: 21.04.2022
(51) Int. Cl.: G16B 20/20, G16B 40/20

(54) **DETECTING LOSS OF HETEROZYGOSITY IN HLA ALLELES USING MACHINE-LEARNING MODELS**
NACHWEIS DES HETEROZYGOTIEVERLUSTES IN HLA-ALLELEN UNTER VERWENDUNG VON MASCHINENLERNMODELLEN
DÉTECTION DE PERTE D'HÉTÉROZYGOSITÉ DANS DES ALLÈLES HLA À L'AIDE DE MODÈLES D'APPRENTISSAGE MACHINE

(30) Priority: 22.04.2021 US 202163178151 P
(43) Date of publication of application: 28.02.2024
(73) Proprietor: Personalis, Inc., Fremont, CA 94555 (US)
(72) Inventor: PYKE, Rachel, Marty, Fremont, CA 94555 (US); MELLACHERUVU, Dattatreya, Fremont, CA 94555 (US); DEA, Steven, Fremont, CA 94555 (US); ABBOTT, Charles, Fremont, CA 94555 (US); ZHANG, Simo, V., Fremont, CA 94555 (US); LEVY, Eric, Fremont, CA 94555 (US); WEST, John, Fremont, CA 94555 (US); CHEN, Richard, Fremont, CA 94555 (US); BOYLE, Sean, Michael, Fremont, CA 94555 (US)
(74) Representative: Secerna LLP
(86) International application number: PCT/US2022/025752
(87) International publication number: WO 2022/226186

(56) References cited:
- WO-A1-2020/168008
- US-A1- 2020 258 597

## Description

### BACKGROUND OF THE INVENTION

Immune checkpoints generally refer to a set of inhibitory pathways hardwired into the immune system, which regulate the duration and amplitude of physiological immune responses. When activated, the immune checkpoint molecules (e.g., PD-1) suppresses the immune system in order to prevent it from attacking cells indiscriminately. Although immune checkpoints are generally effective, tumor cells may manipulate such mechanism to prevent the immune system from eliminating tumor cells.

Immune checkpoint blockade therapy is a recent treatment to counter the mechanism of tumor cells. Immune checkpoint blockade therapies use medications such as immune checkpoint inhibitors to activate the immune system to recognize and eliminate cancerous cells. The immune checkpoint blockade therapies enable the immune system to properly recognize and eliminate tumor cells that present neoantigens via major histocompatibility complexes (MHC). Despite this early success, a large percentage of subjects do not respond to these therapies, due to complex tumor intrinsic and extrinsic mechanisms of tumor cells to resist and evade immune checkpoint blockade therapies. Elucidating the cause of such immune checkpoint blockade resistance has proven to be more challenging than initially anticipated.

One of the mechanisms causing immune checkpoint blockade resistance may include loss of heterozygosity in human leukocyte antigen (HLA) genes. A neoantigen corresponding to a mutated gene of a tumor cell can bind to a HLA protein encoded by a particular HLA allele and be presented on the cell surface. When the presented neoantigen is detected, the immune system can respond by deploying T cells that identify and eliminate the tumor cell by detecting the presented neoantigen. Thus, effectiveness of the immune system may depend on whether the neoantigen is presented on the tumor cell surface. Conversely, preventing the presentation of neoantigens can result in the T cells being unable to detect the corresponding tumor cells.

Various studies suggest that tumor cells often have loss of heterozygosity in HLA genes, such that the corresponding HLA proteins of the deleted HLA alleles are not available to present the neoantigens on tumor cell surfaces. For example, each human subject has six different HLA alleles capable of presenting a diverse set of antigens to the immune system. The germline sequence diversity of HLA alleles can impact tumor evolution by mediating the presentation of neoantigens to the immune system. This impact of HLA sequence diversity appears to be more pronounced in the presence of the immune checkpoint blockade therapies. As tumor cells mutate, somatic loss of heterozygosity in the HLA allelic regions can occur, thereby causing reduction in HLA sequence diversity. Such loss of heterozygosity of HLA alleles is increasingly being recognized as a cause of immune checkpoint blockade resistance by tumor cells.

Thus, detecting loss of heterozygosity of HLA alleles from sequencing data can be beneficial in anticipating immune checkpoint blockade resistance and developing a corresponding therapy for a given subject. However, conventional techniques can be deficient in accurately detecting loss of heterozygosity of HLA alleles. For example, a conventional technique for detecting HLA loss of heterozygosity can include performing a genome-wide interrogation for detecting copy numbers. In this technique, a decrease of copy numbers around the HLA genes may indicate its loss of heterozygosity. This conventional technique, however, can be unreliable in detecting HLA loss of heterozygosity from sequencing data, for at least the following reasons. First, the polymorphic nature of the mutated genes causes poor alignment of corresponding sequence reads to the reference genome. Second, the complexity of the sequence variation can obscure the specific HLA allele that has been deleted, which is information crucial for neoantigen therapy design.

Another conventional technique can include identifying copy number variations of HLA genes after alignment of sequence data to HLA allele-specific reference sequences. However, most allele-specific alignment techniques relied on by the conventional copy number variant algorithms fail to account for HLA-specific challenges such as differences in exome probe capture between alleles. Moreover, copy number variant algorithms can be notoriously poor for biological samples with low tumor purity and have trouble detecting subclonal deletions, thereby raising concerns regarding the sensitivity and accuracy of these algorithms. Thus, despite growing interest, conventional techniques end up relying on deletions of flanking regions surrounding the HLA allelic region as a proxy for HLA loss of heterozygosity, rather than developing an HLA loss of heterozygosity specific algorithm. In view of the above, it is challenging to accurately detect HLA loss of heterozygosity.

Moreover, validating performance of HLA loss of heterozygosity detection algorithms has been an additional challenge in the field. For example, a conventional technique includes assessing concordance between HLA loss of heterozygosity calls and copy number calls made by a standard CNV algorithm in regions flanking each HLA gene. Another conventional technique includes designing primers to capture the regions surrounding the HLA genes applying PCR to identify copy number loss of HLA alleles in subjects. However, neither of these approaches validates the identity of specific HLA allele that can be lost nor addresses the accuracy of calls for low tumor purity samples or samples with HLA loss of heterozygosity subclonality.
Document US 2020/258597 A1 discloses a method for HLA typing of sequence data of a subject, as well as detecting loss of heterozygosity (LOH) using a trained model for determining the LOH status of an HLA allele.

### SUMMARY OF THE INVENTION

The invention is set out in the appended claims.

### SUMMARY OF RELATED DISCLOSURE

In some embodiments, a method of detecting loss of heterozygosity in HLA alleles is provided. The method can include accessing a trained machine-learning model, which was trained using a training data set that included one or more sets of training features corresponding to an HLA allele identified in a tumor sample corresponding to a subject of a set of subjects. A first set of training features includes, for a genomic region of the HLA allele: (i) an adjusted B allele frequency that represents a ratio between a first B allele frequency of heterozygous alleles in the tumor sample that correspond to the genomic region and a second B allele frequency of heterozygous alleles in the genomic region and associated with one or more control samples; and (ii) a ratio between a first allele-specific coverage of the tumor sample that corresponds to the genomic region and a second allele-specific coverage of the one or more control samples that corresponds to the genomic region. A second set of training features includes, for the HLA allele, an indication of whether at least part of a flanking genomic region surrounding the HLA allele has been deleted.

The method can also include receiving sequence data corresponding to a biological sample of a particular subject. The method can also include using the machine-learning model to generate a result corresponding to a probability of whether a loss of heterozygosity exists in an HLA allele identified in the biological sample of the particular subject by processing the sequence data using the machine-learning model. The method can also include outputting the result.

Some embodiments of the present disclosure include a system including one or more data processors. In some embodiments, the system includes a non-transitory computer readable storage medium containing instructions which, when executed on the one or more data processors, cause the one or more data processors to perform part or all of one or more methods and/or part or all of one or more processes disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention as claimed has been specifically disclosed by some embodiments and optional features, modification and variation of the concepts herein disclosed may be resorted to by those skilled in the art, and that such modifications and variations are considered to be within the scope of this invention as defined by the appended claims.

### BRIEF DESCRIPTION OF THE DRAWINGS

The present disclosure is described in conjunction with the appended figures:
FIG. 1 illustrates a schematic diagram for preparing sequence alignment data for detecting loss of heterozygosity in HLA alleles, according to some embodiments.
FIG. 2 illustrates a diagram corresponding an example set of features of a training data set for training a machine-learning model to detect loss of heterozygosity in HLA alleles, according to some embodiments.
FIG. 3 illustrates various features identified from a training data set for training a machine-learning model to detect loss of heterozygosity in HLA alleles, according to some embodiments.
FIG. 4 shows example data generated by a machine-learning model trained to detect loss of heterozygosity in HLA alleles, according to some embodiments.
FIG. 5 shows an example of cell line based limit of detection technique for validating a machine-learning model trained to detect loss of heterozygosity in HLA alleles, according to some embodiments.
FIG. 6 shows an example set of results representing performance levels of the DASH model based on an *in silico* cell line based limit of detection analysis.
FIG. 7 shows an example of allele-specific genomic technique for validating a machine-learning model trained to detect loss of heterozygosity in HLA alleles, according to some embodiments.
FIG. 8 shows example set of results representing performance levels of the DASH model based on allele-specific genomic validation with digital PCR.
FIG. 9 shows a schematic overview of using immunopeptidomic data for validating a machine-learning model trained to detect loss of heterozygosity in HLA alleles, according to some embodiments.
FIG. 10 shows an example of quantitative immunopeptidomics data corresponding to a relationship between control samples without predicted HLA loss of heterozygosity and samples with predicted HLA loss of heterozygosity, according to some embodiments.
FIG. 11 shows an example of quantitative immunopeptidomics data corresponding to control samples without predicted HLA loss of heterozygosity, according to some embodiments.
FIG. 12 shows an example of quantitative immunopeptidomics data corresponding to samples with predicted HLA loss of heterozygosity, according to some embodiments.
FIG. 13 shows HLA loss of heterozygosity predicted across tumor types, according some embodiments.
FIG. 14 shows a set of experimental results 1400 that show relationship between HLA loss of heterozygosity and antigen presentation across various tumor types, according to some embodiments.
FIG. 15 shows experiment data that identify neoantigen expansion in response to immune checkpoint inhibitor therapy on head and neck squamous cell carcinoma subjects, according to some embodiments.
FIG. 16 shows additional experiment data corresponding to HLA loss of heterozygosity on tumors treated with immunotherapy, according to some embodiments.
FIG. 17 includes a flowchart illustrating an example of a method of predicting loss of heterozygosity in HLA alleles, according to certain some embodiments.
FIG. 18 illustrates an example of a computer system for implementing some embodiments disclosed herein.

### DETAILED DESCRIPTION OF THE INVENTION

### I. Overview

As described above, accurate detection of HLA loss of heterozygosity can significantly improve accuracy and effectiveness of cancer immunotherapies, including immune checkpoint blockade therapies. Although allele-specific alignment techniques may be an improvement over other genomic interrogation techniques, variability in exome capture across alleles and the relatively short sequences of the HLA genes introduce additional challenges for identifying changes in copy number. Moreover, conventional techniques cannot accurately and comprehensively determine the limit of detection, sensitivity, and specificity of HLA loss of heterozygosity detection algorithms.

To address at least the above deficiencies of conventional systems, the present techniques can be used, the present techniques can use a machine-learning approach to detect loss of heterozygosity in HLA alleles. A machine learning model for identifying deletion of allele-specific HLAs ("the DASH model") can be accessed. In particular, the DASH model can be trained using training data that include, for a subject of a set of subjects, the following features: (1) allele-specific features; (2) subject-specific features; and (3) whole-exome features. The allele-specific features can include, for a genomic region of an HLA allele: an adjusted B allele frequency that represents a ratio between a first B allele frequency of heterozygous alleles in the tumor sample that correspond to the genomic region and a second B allele frequency of heterozygous alleles in the genomic region and associated with one or more control samples; and a ratio between a first allele-specific coverage of the tumor sample that corresponds to the genomic region and a second allele-specific coverage of the one or more control samples that corresponds to the genomic region. By collectively using the above training features, the DASH model can be trained to accurately detect loss of heterozygosity in HLA alleles. In some instances, the allele-specific features correspond to a genomic region of an HLA allele that was identified as having a somatic mutation.

As referred herein, a B allele frequency is a normalized measure of the allelic intensity ratio of two alleles (A and B), such that a B allele frequency of 1 or 0 indicates the complete absence of one of the two alleles (e.g. AA or BB), and a B allele frequency of 0.5 indicates the equal presence of both alleles (e.g. AB). For example, a first B allele frequency can indicate, for a given genomic position, an allelic intensity ratio between HLA-B*46:01:01 and HLA-B*13:01:01 that corresponds to a normal biological sample. A second B allele frequency can indicate, for the same genomic position, an allelic intensity ratio between HLA-B*46:01:01 and HLA-B*13:01:01 that corresponds to a tumor sample. The adjusted B allele frequency can be a ratio determined by dividing the first B allele frequency with the second B allele frequency (or vice versa).

The subject-specific features can include an estimated tumor purity value and an estimated tumor ploidy value corresponding to a tumor sample of the subject. Tumor purity, as used herein, refers to as a ratio of tumor cells to total cells in the sample. Tumor ploidy, as used herein, refers to an average copy number of the entire tumor genome. The whole-exome features can include, for the HLA allele, an indication of whether at least part of a flanking genomic region surrounding the HLA allele has been deleted.

The DASH model trained with the above training features may then be used to process sequence data and generate a result corresponding to a probability of whether a loss of heterozygosity exists in an HLA allele identified in the biological sample of the particular subject. The sequence data corresponding to a biological sample of a particular subject can be accessed. As used herein, sequence data refers to data corresponding to a biological sequence corresponding to nucleic acid (*e.g.,* DNA, RNA) or protein (*e.g.,* alanine arginine). In some instances, sequence data includes one or more sequence reads. The sequence data can be generated by using whole genome sequencing or whole exome sequencing on the biological sample to generate a plurality of sequence reads. After the sequence data is generated, one or more HLA alleles can be identified from the sequence data.

Reference sequences corresponding to the identified HLA alleles can be retrieved, and the sequence reads can be aligned to the retrieved reference sequences. After alignment, allele-specific data for each of the identified HLA alleles corresponding to the sequence data can be identified. In some instances, the allele-specific data identifies a number of sequence reads that align to each genomic region corresponding to the identified HLA alleles.

The trained DASH model uses the allele-specific data for each of the identified HLA alleles as an input to generate the result. Other types of information corresponding to the identified HLA alleles (*e.g.,* an indication of whether at least part of a flanking genomic region surrounding the HLA allele has been deleted) can be used as additional input to the trained DASH model. The trained DASH model that includes one or more gradient boosting algorithms can process the above features of the sequence data to generate the result. In some instances, the result is used to predict a decrease in efficacy of an immune checkpoint blockade therapy being administered to the particular subject.

Accordingly, some embodiments of the present disclosure provide a technical advantage over conventional systems by accurately detecting loss of heterozygosity in HLA alleles. For example, the DASH model can accurately detect loss of heterozygosity in HLA alleles by processing whole-exome sequencing data, which differs from conventional techniques that rely on sequence reads corresponding to the HLA alleles only. Taking into account that the HLA genes are relatively short and most deletions involve much larger genomic regions, the DASH model uses the entire whole-exome platform to incorporate sequence information from around the HLA genes as well inside them. As a result, the detection accuracy of the DASH model can be validated with sensitivity levels at 100% for samples having tumor purity levels above 8% and specificity levels at 100% for samples across all tumor purity levels. Thus, the accurate detection of HLA loss of heterozygosity facilitates investigation of tumor cell mechanisms contributing immune checkpoint blockade resistance and development of new cancer immunotherapies. Moreover, some embodiments of the present disclosure can use allele-specific features to accurately identify which genomic region of the HLA allele can be deleted, thereby detecting loss of heterozygosity with increased granularity.

The following examples are provided to introduce certain embodiments. In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, and techniques may be shown without necessary detail in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as an "example" is not necessarily to be construed as preferred or advantageous over other embodiments or designs.

### II. Sequence Data and Allele-Specific Coverages of HLA Alleles

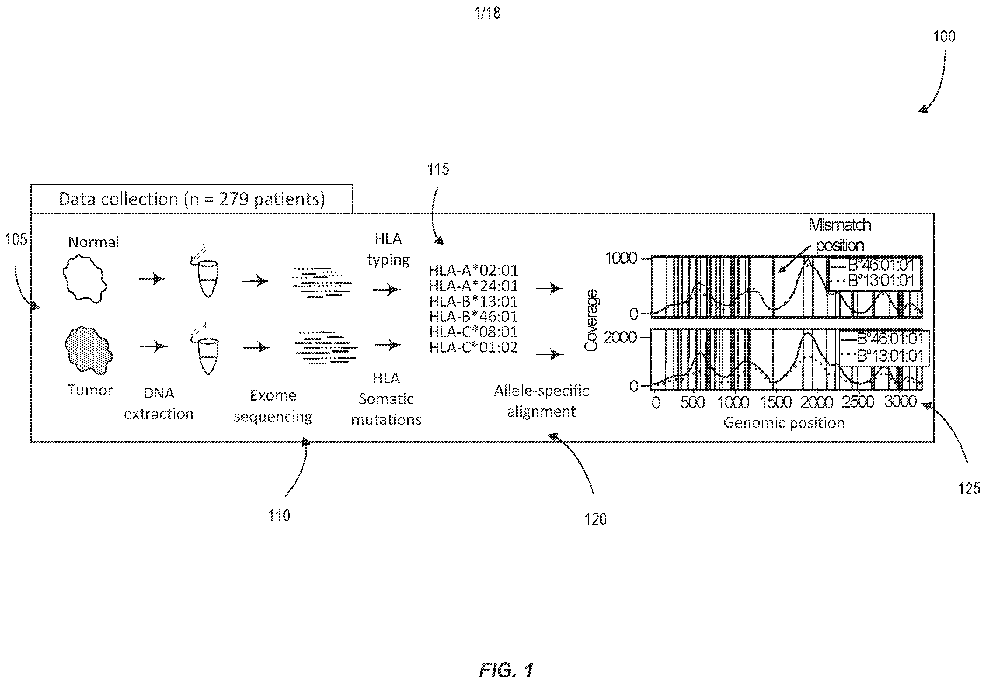

FIG. 1 illustrates a schematic diagram 100 for preparing sequence alignment data for detecting loss of heterozygosity in HLA alleles, according to some embodiments. At step 105, tumor and normal biological samples corresponding to each of a set of subjects can be collected. For example, each of the tumor and normal sample pair can be a tissue sample (e.g., adjacent normal sample) or a blood/plasma sample of a corresponding subject. In some instances, a subset of samples are collected from different tumor types. For each subject, paired formalin-fixed, paraffin-embedded (PFEE) or fresh frozen samples can be profiled to identify various characteristics corresponding to the normal and tumor samples. For example, the characteristics may include comprehensive tumor mutation information, gene expression quantification, neoantigen characterization, HLA alleles (types and mutations), and tumor microenvironment profiling.

At step 110, whole exome library preparation and sequencing can be executed to generate sequence reads corresponding to each biological sample. In some instances, whole genome sequencing is performed to generate the sequence reads. DNA from tumor and peripheral blood mononuclear cells/adjacent samples can be used to construct whole-exome capture libraries, in which the libraries are built based on two whole-exome sequencing (WES) capture kits: Agilent SureSelect Human All Exon v5 plus untranslated regions and Agilent SureSelect Clinical Research Exome. Modifications can be made to sequencing protocols to yield an approximately 250 bp average library insert size (for example). In some instances, various polymerases are used to generate the sequence reads, including a KAPA HiFi DNA Polymerase and Herculase II DNA polymerase (for example). Sequencing can be performed at 20G sequencing depth for normal samples and 35G sequencing depth for tumor samples. Using the above example sequencing methods, over >300x coverage exome-wide can be available for >20,000 genes, and >1000x coverage can be available for boosted regions including over 500 cancer-associated genes, including HLA-A, -B and -C alleles.

At step 115, HLA genotyping (alternatively referred to as "HLA typing") can performed to identify one or more HLA alleles from the sequence reads. HLA types can be calculated up to 6 digits. In some instances, the sequence reads corresponding tumor samples are processed to identify somatic mutations corresponding to the one or more HLA alleles. In some instances, additional types of data can be identified from the tumor and normal samples. For example, the sequencing data can be analyzed to identify allele-specific copy number alterations from the particular HLA allele-type. Additionally or alternatively, the sequencing data can be analyzed to estimate tumor purity (alternatively referred to as tumor cellularity) and tumor ploidy.

At step 120, the sequence reads can be aligned to one or more reference sequences (e.g., a hs37d5 reference genome build). The subject-specific homologous alleles can be aligned to determine positions of difference between the alleles. Both single nucleotide variants (SNVs) and indels can be detected in the alignment. In some instances, only the first position of each indel can be considered to ensure SNVs can be appropriately weighted. HLA alleles with fewer than 5 positions of difference between them can be considered to be homozygous.

In some instances, the reference sequences corresponding to the identified HLA alleles are retrieved by querying an HLA-allele database. The HLA-allele database can retrieve the reference sequences corresponding to the identified HLA alleles using an imputation approach. The HLA-allele database can be initialized with a particular format, such as multiple sequence alignment (MSA) format from IMGTv312. To implement imputation, cDNA data can be used to impute exons in HLA alleles and incompletely sequenced HLA alleles with a reference allele that had protein-level identity as defined by an identical 4 digit nomenclature. In the event that no such allele exists, a reference sequence from the same HLA subtype can be retrieved from the HLA-allele database based on identical 2 digit nomenclature. If there are multiple options with identical 2 digit nomenclature, the first allele listed in the MSA can be used. To impute the intronic regions of each allele, the above approach can be taken using a gDNA file. The full length genomic sequences of each allele can thus be imputed by assembling exons from the cDNA imputation set and the introns from the gDNA imputation step. In some instances, duplicate reads are removed. Additionally or alternatively, the Genome Analysis toolkit (GATK) can be used to correct base quality scores and improve sequence alignment of the sequence reads.

In some instances, any sequence reads that had soft clipping for more than 20% of their total length are excluded. Any reads that contained mismatches can be discarded in order to improve quality of coverage information. However, if a somatic mutation within the HLA alleles is identified, the stringency can be lifted to allow sequence reads with a single mismatch.

At step 125, allele-specific data of identified HLA alleles corresponding to each of the normal and tumor samples can be determined. The allele-specific data can identify a number of sequence reads that align to each genomic region corresponding to the identified HLA alleles. A copy number alteration of sequence reads for a particular genomic position may indicate a loss of heterozygosity of the corresponding HLA allele. For example, with respect to B*13:01:01 HLA allele, a decreased number of sequence reads can be identified in genomic positions ranging between 1800-2000 in a tumor sample relative to the normal sample. Such copy number alteration can indicate to a loss of heterozygosity of the B*13:01:01 HLA allele.

### III. Machine-learning Models for Predicting Loss of Heterozygosity in HLA Alleles

### (a) Overview

As described above, the DASH model can be used to process sequence data and generate a result corresponding to a probability of whether a loss of heterozygosity exists in an HLA allele identified in the biological sample of the particular subject. To initiate the process, the sequence data corresponding to a biological sample of a particular subject can be generated by using whole genome sequencing or whole exome sequencing on the biological sample. The biological sample can be a tissue sample that may include DNA derived from tumor or healthy cells. In some instances, the biological sample includes cell-free DNA, some of which can have originated from healthy cells and some from tumor cells.

In some instances, HLA genotyping is performed on the plurality of sequence reads to identify one or more HLA alleles that correspond to the sequence data. Reference sequences corresponding to the identified HLA alleles can be retrieved, and the sequence reads can be aligned to the retrieved reference sequences. After alignment, allele-specific coverage for each genomic region can be determined for the identified HLA alleles corresponding to the sequence data. In some instances, allele-specific coverage identifies a number of sequence reads that align to each genomic position of the identified HLA allele.

The trained DASH model can be used to process the allele-specific data for each of the identified HLA alleles to generate the result corresponding to a probability of whether a loss of heterozygosity exists in each of the identified HLA alleles. Other types of information corresponding to the identified HLA alleles (*e.g.,* an indication of whether at least part of a flanking genomic region surrounding the HLA allele has been deleted) can be used as input to the trained DASH model. The trained DASH model that includes one or more gradient boosting algorithms can process the above features of the sequence data to generate the result.

In some instances, the allele-specific data for each of the identified HLA alleles is as an input to the trained DASH model. Other types of information corresponding to the identified HLA alleles (*e.g.,* an indication of whether at least part of a flanking genomic region surrounding the HLA allele has been deleted) can be used as input to the trained DASH model. The trained DASH model that includes one or more gradient boosting algorithms can process the above features of the sequence data to generate the result. In some instances, the result is used to predict a decrease in efficacy of an immune checkpoint blockade therapy being administered to the particular subject.

The DASH model can be trained using the aligned sequence data and allele-specific coverages to accurately detect allele-specific loss of heterozygosity in HLA genes. Specifically, a training dataset for the DASH model can include features derived from paired tumor and normal samples (either adjacent tissue or peripheral blood mononuclear cells) from subjects. As described above, to identify the training features for the training data set, aligned sequence data and allele-specific coverages corresponding to HLA alleles can be identified by applying whole exome sequencing to paired tumor and normal samples corresponding to the subject.

In some instances, capture probes covering specific HLA alleles are applied in addition to the whole exome sequencing. Sequence reads corresponding the HLA alleles for each subject can be mapped to each subject-specific HLA reference. From the alignment data, allele-specific coverages can be determined for each genomic region and training features corresponding to somatic variants of the HLA alleles can be identified. The training features can include a modified B allele frequency that accounts for differences in probe capture and consistency of allele-specific coverages across various HLA alleles. In some instances, the training features further include information corresponding to genomic regions surrounding the HLA alleles as a vast majority of HLA loss of heterozygosity events are large deletions.

### (b) Model selection

The DASH model can include one or more gradient-boosting algorithms, which can be trained to detect loss of heterozygosity in HLA alleles. Gradient boosting refers to a machine-learning technique for regression and classification problems that produce a prediction model in the form of an ensemble of weak prediction models. The technique may build a model in a stage-wise fashion and generalizes the model by allowing optimization of an arbitrary differentiable loss function. Gradient boosting combines weak learners into a single strong learner in an iterative fashion. As each weak learner is added, a new model is fitted to provide a more accurate estimate of the response variable. The new weak learners can be maximally correlated with the negative gradient of the loss function, associated with the whole ensemble. Examples of the gradient boosting machines can include XGBoost and LightGBM. Additionally or alternatively, other types of machine-learning techniques can be used to build the binding model, including bagging procedures, boosting procedures, and/or random forest algorithms.

### (c) Example training data set

An example training data included a set of 720 heterozygous HLA genes which collected from 279 subjects across multiple tumor types. All features described above for each heterozygous gene were generated and each case of HLA loss of heterozygosity were manually labeled. To train the DASH model, 500 heterozygous genes were separated for training and 220 heterozygous genes were kept separately for testing. With respect to model selection, the DASH model can include a gradient boosting algorithm (e.g., XGBoost) to learn how to detect HLA loss of heterozygosity in each pair of HLA alleles from the features described above. If HLA loss of heterozygosity was detected by the DASH model, the allele with the lower coverage was labeled as deleted. Though rare, few cases with a bi-allelic deletion were detected. If the DASH model detects HLA loss of heterozygosity and the allele with higher coverage has an allele-specific coverage ratio below 0.5 for at least 25% of the bins, both HLA alleles are labeled as deleted.

### (d) Features of the training data set

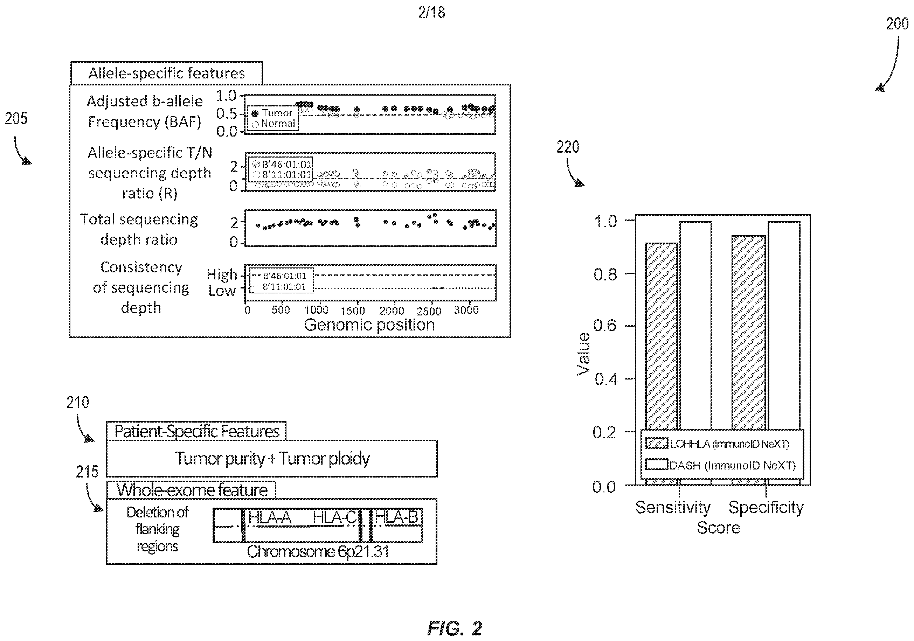

FIG. 2 illustrates a diagram 200 corresponding an example set of training features of a training data set for training a machine-learning model to detect loss of heterozygosity in HLA alleles, according to some embodiments. Sequence alignment data corresponding to normal and tumor samples can be analyzed to identified somatic variants. The diagram 200 illustrates various training features 205, 210, and 215 used to train the DASH model. First, allele-specific features 205 can include the adjusted B allele frequency, coverage ratio between tumor and normal samples, and consistency of coverage. The training features may also include subject-specific features 210, such as tumor purity and tumor ploidy. Finally, whole-exome features 215 can include deletion of flanking regions corresponding to the exomes of the identified HLA allele.

From the allele-specific coverage data, the allele-specific features 205 can be determined. The allele-specific features 205 include the following:
*1. Adjusted B allele frequency:* At each position of mismatch, the B allele frequency can be calculated for the tumor and normal sample separately. Then, the tumor B allele frequency can be divided by the normal B allele frequency. The normal sample can be used to adjust the B allele frequency, because there can be variability in the probe capture of each specific allele. To consolidate the ratios into a single feature, the allele references can be broken into bins of 150 base pairs in length. The absolute value of the median adjusted-B allele frequency can be calculated for each bin. In some instances, the median value across all bins is used as the training feature. The adjusted B allele frequency feature has a lower bound of 0, with larger numbers indicating a higher chance of loss of heterozygosity in the HLA gene.
*2. Allele-specific coverage ratio:* At each position of mismatch between the homologous alleles of tumor and normal samples, a ratio between coverage in the tumor sample and the coverage in the normal sample is calculated for each allele. Each ratio value can be normalized by the exome-wide number of tumor reads divided by the exome-wide number of normal reads. Thus, despite variability in sequencing depth between each run, the expected allele-specific coverage ratio is one if there is no copy number variation. Then for each bin, the median coverage ratio can be calculated for each allele and the lower value amongst the two alleles is considered for that bin. In some instances, the median value across all of the bins is used as the training feature. The allele-specific coverage ratio can have a lower bound of 0, with an expected value of 1 if there is no copy number variation. Lower allele-specific coverage ratios suggest a high probability of loss of heterozygosity in an HLA gene.
*3. Total coverage ratio:* If a heterozygous pair of alleles has a high combined sequencing depth, allelic imbalance may be driven by a large amplification in an allele rather than a deletion in an allele. At each position of mismatch between the homologous alleles, the ratio between coverage in the tumor sample to the coverage in the normal sample is calculated for each allele. Then, the sum of both alleles can be taken as the value representing each bin (e.g., 150-bp bin). Finally, the median across the bins is used as the total coverage ratio feature. The total coverage ratio has a minimum of zero, with higher values tending toward genes without HLA loss of heterozygosity.
*4. Consistency of coverage:* An HLA allele that has consistently lower coverage across all mismatch positions is more likely to be a case of HLA loss of heterozygosity. Thus, each allele can be assigned with value 0 or 1 for each bin if it has lower or higher coverage compared to its homologous allele. If the allelic coverage of a bin cannot be determined (no mismatch sites), each allele is given a value of 0.5. Then, an average of the assigned values can be determined across all bins for each allele and assign the higher average to be the value for the feature. The percentage coverage feature ranges from 0.5 to 1 with values closer to 1 representing a higher likelihood of HLA loss of heterozygosity.

In addition, the subject-specific features 210 can include the following:
*1. Tumor purity:* The tumor purity feature can be estimated. The value ranges from 0.1 to 1, with 0.1 being the least pure tumor and 1 being the most pure tumor. Tumor purity, as used herein, refers to as a ratio of tumor cells to total cells in the sample.
*2. Tumor ploidy:* The tumor ploidy feature can be estimated. The values are whole integers that are greater than or equal to one. Tumor ploidy, as used herein, refers to an average copy number of the entire tumor genome.

Finally, the whole-exome features 215 can include the following:
1. *Deletion of flanking regions*: Since most instances of HLA loss of heterozygosity are due to large deletions, a feature representing deletions in the flanking regions of each HLA gene can be determined to capitalize on information from a larger number of variable sites. The B allele deletion can be called, then a flanking region deletion can be called from the B allele deletion if there is a deletion within 10,000 base pairs in either direction of the HLA gene. This deletion feature can be binary, with 0 representing a deletion.

The diagram 200 additionally shows a bar graph 220 demonstrating that the trained DASH model performs better than other conventional techniques, even when the same sequencing data is used. In this example, biological samples with tumor purity below 20% are removed from the analysis. As shown in the bar graph 220, 100% sensitivity and 99.7% specificity levels of the trained DASH model (shown in green) are respectively greater than 91.8% sensitivity and 94.3% specificity levels of an LOHHLA algorithm (shown in blue), which is an existing conventional technique for detecting loss of heterozygosity as published in McGranahan, Nicholas et al. "Allele-Specific HLA Loss and Immune Escape in Lung Cancer Evolution." Cell vol. 171,6 (2017): 1259-1271.e11. doi:10.1016/j.cell.2017.10.001. When all biological samples are considered (including those with tumor purity that is below 20%), the DASH model reaches 98.7% specificity and 92.9% sensitivity (F-1 Score = 0.939), while the LOHHLA algorithm only achieves 94.3% specificity and 78.8% sensitivity (F-1 Score = 0.777). The DASH model also outperforms other existing conventional techniques. For example, Sequenza detects HLA loss of heterozygosity at 92.9% specificity and 95.0% sensitivity (F1-Score = 0.848). Moreover, none of the above conventional techniques for detecting loss of heterozygosity was able to identify the specific allele that has been lost.

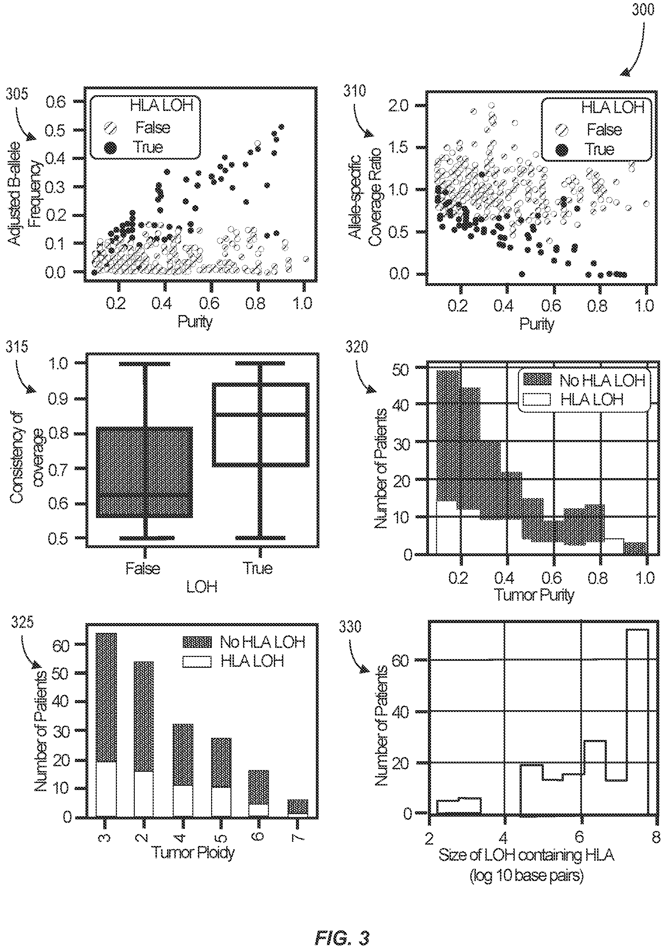

FIG. 3 illustrates additional features identified from a training data set 300 for training a machine-learning model, according to some embodiments. The training data set 300 may include a scatter plot 305 showing the relationship between adjusted B allele frequency and tumor purity. HLA genes with loss of heterozygosity are shown in filled color, and HLA genes without loss of heterozygosity are shown in partially-shaded color. As shown in the scatter plot, a higher adjusted B allele frequency value can be indicative of HLA loss of heterozygosity. In some instances, the adjusted B allele frequency can be determined by dividing the tumor B allele frequency by the adjacent normal B allele frequency for a given genomic region.

The training data set 300 may also include a scatter plot 310 showing a relationship between allele-specific coverage ratio and tumor purity. HLA genes with loss of heterozygosity are shown in filled color and HLA genes without loss of heterozygosity are shown in partially-shaded color. In some instances, allele-specific coverage ratio is determined dividing the tumor coverage of an allele by the adjacent normal coverage of the same allele and normalizing by the coverage across the rest of the exome.

The training data set 300 may also include boxplots 315 showing the difference in distribution between consistency in coverage for subjects with and without HLA loss of heterozygosity. The boxplots 315 can be used to capture an observation that alleles with consistently lower coverage than the alternate allele across the entire gene is likely to be deleted, whereas sporadically lower coverage may be due to stochastic variation (p = 2.2e-14, paired T test). In addition, a difference of distribution can be shown between total sequencing depth for subjects with and without loss of heterozygosity. As noted above, the total coverage for subjects with HLA loss of heterozygosity is relatively lower than the total coverage for subjects without HLA loss of heterozygosity. Thus, in order to distinguish allelic imbalance driven by loss of heterozygosity from allelic imbalance driven by a large amplification of the alternate allele, total coverage ratio, that captures the combined coverage of the two alleles. Subjects with HLA loss of heterozygosity have significantly lower total coverage ratios (p=0.0004, paired T test) with the null hypothesis that subjects with and without HLA loss of heterozygosity will have the same distribution of total coverage ratios.

The training data set 300 may also include histograms 320 and 325, in which the histogram 320 shows the distributions of tumor purity for subjects with and without loss of heterozygosity and the histogram 325 shows distribution of tumor ploidy for subjects with and without loss of heterozygosity. HLA genes with loss of heterozygosity are shown in transparent color, and HLA genes without loss of heterozygosity are shown in shaded color. The histograms 320 and 325 showed that tumor purity and tumor ploidy are identical across HLA-A, HLA-B and HLA-C of a particular subject.

The training data set 300 can also include a histogram 330 that shows a distribution of loss of heterozygosity sizes corresponding to HLA genes across all subjects. Since 73% of copy number alterations causing HLA loss of heterozygosity are deletions of greater than one megabase, genomic regions flanking the genes of interest can provide useful information to supplement the within-gene data. Thus, the whole exome nature of the training data set 300 was used to generate a feature corresponding to deletion of flanking regions, which can measure deletions in the 10 kb region surrounding each HLA gene.

### (e) Training process

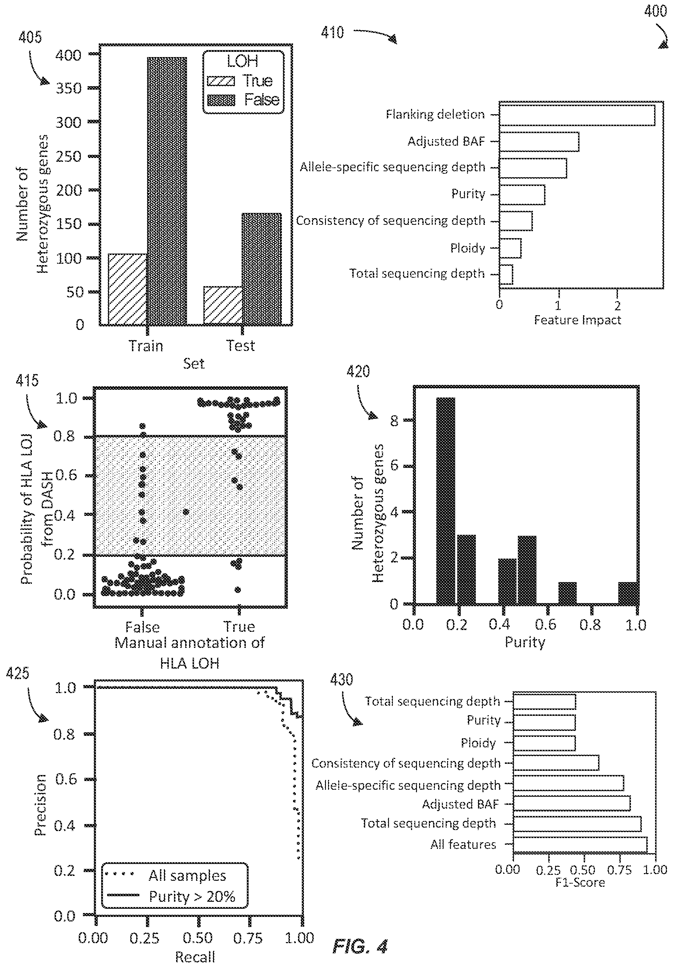

FIG. 4 shows example data 400 generated by a machine-learning model trained to detect loss of heterozygosity in HLA alleles, according to some embodiments. The example data 400 shows a bar plot 405 showing a number of heterozygous gene pairs with and without HLA loss of heterozygosity in the training and test set for the DASH model. For the example training process, six features for 720 heterozygous alleles were collected from 279 subjects. Homozygous alleles were excluded because loss of heterozygosity cannot occur on homozygous alleles. Training labels were added to 720 alleles and deleted alleles were manually curated. The example data were split into a training data set (n=500, ~70%) and a test data set (n=220, ~30%), with roughly equivalent class distribution. The DASH model that includes a gradient boosted regression (XGBoost) algorithm was trained to predict deleted HLA alleles.

A bar plot 410 shows an impact of each feature in the trained DASH model for detecting loss of heterozygosity in HLA alleles. In some instances, the impact of each feature is measured based on a game theory model. The bar plot 410 revealed that all six of our features were independently contributing to the DASH model, with deletion of flanking regions and adjusted B-allele frequency impacting the outcome most significantly relative to other features (e.g., tumor ploidy).

A scatter plot 415 shows a distribution of probabilities of HLA loss of heterozygosity returned by the trained DASH model for biological samples corresponding to test dataset and manually annotated as having or not having loss of heterozygosity in HLA alleles. In, the scatter plot 415, a shaded region indicates ambiguous calls by the DASH model. Since the XGBoost algorithm returns a continuous metric, the HLA loss of heterozygosity calls were divided into high and low confidence calls. In the test data set, the trained DASH model reaches 96% specificity and 89% sensitivity when considering high confidence calls (> 0.8 loss of heterozygosity prediction cutoff). As noted above, the DASH model can perform at higher specificity and sensitivity levels over other conventional techniques. For comparison, the LOHHLA algorithm reaches 92% specificity and 76% sensitivity using the same test data set.

A histogram 420 shows a distribution of tumor purities of HLA genes in which ambiguous (>0.2 and <0.8) calls were made by the DASH model. As shown in the histogram 420, the majority of the borderline and incorrect calls have low tumor purity, highlighting the difficulty of accurately predicting HLA loss of heterozygosity at low tumor purity levels. When samples with tumor purity below 20% are removed the test data set, the performance level of the DASH model increases, as shown in a precision recall curve 425.

The precision recall curve 425 shows a performance level of the DASH model on a held out dataset (n=220 heterozygous genes). In the curve 425, the dotted line indicates the performance of all samples, and the solid line indicates the performance of samples with at least 20% tumor purity. When samples with tumor purity below 20% are removed from the test data set, the DASH model reached 97% specificity and 95% sensitivity at a >0.8 loss of heterozygosity prediction cutoff. Continuing with the above example, the DASH model performs better than the LOHHLA algorithm, which achieves 94% specificity and 82% sensitivity over the same test data set that excludes the samples having less than 20% tumor purity. In order to achieve an optimal balance between sensitivity and specificity, the 0.2 threshold was applied for the remainder of the analyses. Using this threshold, a very strong performance of the DASH model was observed on high purity samples (F1-Score=0.93) and poorer performance with the inclusion of low purity samples (F1-Score=0.87) on the test data set.

Finally, a bar plot 430 comparing the F1-Scores of various the DASH models trained on individual features to the DASH model trained on all features of the training data set (see FIG. 2). As shown in the bar plot 430, the flanking regions and the adjusted b-allele frequency generated higher F-1 scores for predicting loss of heterozygosity in HLA alleles. Nonetheless, it was found that none of the features alone could achieve the level of performance of the DASH model that was trained with all features of the training data set.

### IV. Validation Techniques for Predicting Loss of Heterozygosity in HLA Alleles

HLA loss of heterozygosity has been observed as occurring late in tumor progression as a resistance mechanism. Furthermore, tumor types that tend to be most responsive to immune checkpoint blockade (lung, skin) also tend to produce lower purity samples. Thus, a limit of detection analysis with a gold standard sample in both low clonality and low purity settings was used to accurately validate performance of the DASH model.

### (a) Cell line based limit of detection analysis

With respect to limit of detection, a tumor-normal paired lymphoblast cell line sample (NCI-H2009) can be used to assess the DASH model across varying tumor purities and clonalities. In the NCI-H2009 sample, HLA-A is homozygous while: (i) both HLA-B*51:01 and HLA-C*15:02 alleles are deleted; (ii) HLA-B*07:02 and HLA-C*07:02 are retained. Deep sequencing can be performed on the tumor and normal cell lines at 50x coverage and 30x coverage, respectively. To stimulate a realistic sequencing depth, the normal data can be downsampled to reflect 25x sequencing coverage. To create tumor data of decreasing purity, increasing proportions of normal reads can be mixed with decreasing proportions of tumor reads. The combined normal and tumor reads can be summed to an average of 35x sequencing coverage to represent the tumor sample. As used herein, sequencing coverage refers to the average number of reads that align to known reference bases. During sequencing, the sequencing coverage level can be used determines whether variant discovery can be made with a certain degree of confidence at particular base positions. For example, a recommended sequencing coverage for whole-genome sequencing may range between 30x to 50x, depending on application and statistical model. In another example, a recommended sequencing coverage for whole-exome sequencing may be 100x.

All combinations of normal and tumor sub samples can be performed in replicates of 10 using the seqkit library. In some instances, to simulate lower sub clonality, the proportion of tumor reads in the mixture was used as the product of desired tumor purity and sub clonality. The tumor purity can be then inflated to reflect the desired tumor purity. Samples without HLA loss of heterozygosity can be simulated by only including normal reads in the tumor sample and increasing the estimated tumor purity to reflect the desired range. These runs can be used to estimate specificity.

### (b) Allele-specific digital PCR validation

To validate allele-specific HLA loss of heterozygosity in samples, subject-specific primers and probes can be designed and tested for depletion of allele-specific DNA with digital PCR. Since each subject has a unique set of up to 6 HLA class I alleles, subject-specific primers and probes can be designed for each subject. These primers and probes can bind with high specificity to each allele of interest and discriminate against all other alleles and the rest of the genome. Due to the similarity of some homologous alleles, good primers and probes may not exist for all subjects. In some instances, primers and probes are designed for eleven homologous allele pairs with HLA loss of heterozygosity predicted by the DASH model from ten different subjects and one cell line to maximize discrimination between alleles. Furthermore, a probe targeting RNase P can be also used to serve as an internal positive control. The HLA allele and RNase P probes can be assigned different fluorescence to allow multiplexing. A negative control sample (*e.g.,* H₂O) can be used.

To assess the efficiency of the primers and probes, digital PCR can be performed in triplicate on the DNA from the normal and tumor samples (excluding subject C, which can be performed in duplicate). Three samples can be from the training dataset (B, D, K) and the remaining seven samples can be independent. To analyze the data, both the lost and retained will be normalized by the control gene to account for sample input variation. The primers and probes can be deemed successful if the ratio of the HLA allele copies to the multiplexed RNase P copies can be 0.5 in the normal sample because the HLA allele can be expected to be haploid and RNase P is expected to be diploid. Then, for the primer designs that fit this requirement, the allele to RNase P ratio in the tumor DNA is compared to the allele to RNase P ratio in the normal DNA with a one-sided T-test to determine if there has been a significant drop in the tumor. This test is performed for both the predicted retained allele and the predicted lost allele. Allelic imbalance is determined by measuring a significant difference between the predicted lost and predicted retained alleles in the normal DNA and the tumor DNA. Of note, this validation focuses on specific sections of each gene. Thus, it is not formulated to catch small focal deletions in a small portion of the gene.

### (c) Quantitative immunopeptidomics validation

To assess the functional impact of HLA loss of heterozygosity on peptide presentation by MHC molecules, quantitative immunopeptidomics can be performed on two colorectal and four lung tumor-normal paired fresh frozen samples. The samples can be homogenized, normalized for protein content between the tumor and normal and the clarified homogenates can be applied to a pan-MHC-I antibody (W6/32)-linked immunoaffinity resin. In some instances, the success of immunoprecipitation from the lysates is assessed using ELISA, by comparing the MHC concentration pre- and post- IP. MHC-associated peptides can be eluted and collected. Eluted peptides from tumor and normal samples can be labeled and analyzed in a single run for each pair, in high resolution HCD mode.

The resulting raw files of all six samples can be processed together. Peptide identification can be performed using a *de novo* identification followed by a database search. For example, parameters for database search can be as follows -- precursor mass tolerance: 10 ppm, fragment mass tolerance: 0.03 Da, protein database: uniprot sequences downloaded in April 2019, enzyme digestion: none, fixed modifications: carbamidomethylation of cysteine (+57.02 Da) and TMT10plex at all N-terminal amino acids and lysines (+2291.6), variable modifications: protein N-terminal acetylation (+42.0106) and oxidation of Methionine (+15.9949). Peptides can be filtered at 1% FDR and reporter ions can be quantified. The list of quantified peptides can be further filtered to increase the quality of calls by removing peptides that do not have expected TMT n-terminal or lysine modifications, peptides with low intensity (less 10E4 precursor ion intensity) and suspicious peptides with poly amino acids. Then, the intensities can be log2 transformed and the data can be median normalized. Finally, a fold change can be calculated from the log2 transformation, with values less than 0 representing a depletion of peptide in the tumor sample and values greater than 0 representing enrichment of peptide in the tumor sample.

To assess overall changes in presentation between the normal and tumor samples, the absolute values corresponding to the logarithm of the fold changes were compared amongst the samples. Subsequently, the peptide change for specific alleles was estimated. For each subject, each peptide of a peptide set was assigned to an MHC allele. If a peptide can be predicted to bind to multiple alleles, it was considered ambiguous and excluded from the analysis. If the only peptides predicted to bind to an allele can be predicted to bind to more than one allele, they were included but was marked (e.g., an asterisk). The logarithm of the fold change can be visualized to assess the enrichment or depletion of peptides from particular alleles in the tumor sample. The log2 transformed intensity values can be compared with a Wilcoxon Rank Sum Test to assess the statistical significance of any enrichment or depletion. All comparisons with tumor purity use the tumor purity as estimated.

### (d) Predicting HLA-associated neoantigens

The MHC class I presentation prediction model can be performed using a machine-learning model trained using large scale immunopeptidome datasets and benchmarked against a existing binding-prediction model (e.g., NetMHCpan 4.0) with superior performance across several metrics. The output of the trained model can be normalized by allele in a similar manner to NetMHCpan 4.0, creating a rank metric. In some instances, the percentile rank threshold used is 0.1%. All peptide-allele combinations with ranks below this threshold can be considered as peptides that are bound and presented on a cell surface.

### (e) Validation results

### (1) Validation results for cell line based limit of detection analysis

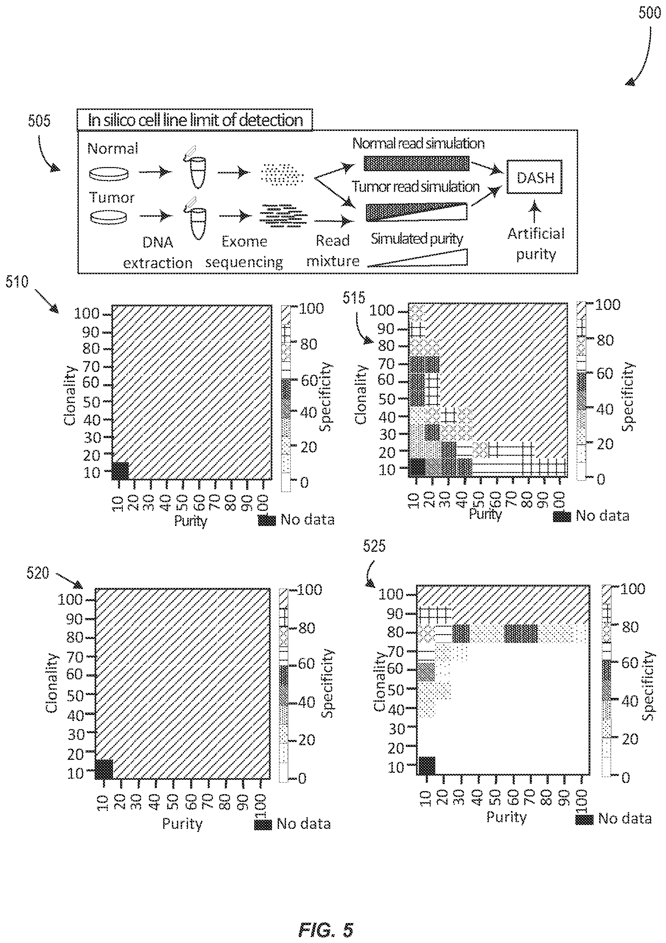

FIG. 5 shows an example of cell line based limit of detection technique 500 for validating a machine-learning model trained to detect loss of heterozygosity in HLA alleles, according to some embodiments. In some instances, *in silico* cell line mixtures and allele-specific genomic validation were used to validate the DASH model. A schematic diagram 505 illustrates an example of mixing tumor and normal cell lines for simulating low purity sample pairs. A heatmap 510 shows specificity levels and a heatmap 515 shows sensitivity level of the DASH model to capture HLA loss of heterozygosity in simulated samples of differing purity and clonality. For the heatmaps 510 and 515, boxes are shaded differently to indicate various sensitivity levels, and grey box indicates no data available. Thus, the heatmaps 510 and 515 show a range of ratios of reads to simulate the potential spectrum of tumor purities and sub-clonalities. As expected, sensitivity levels decreased with lower purity and clonality. However, the DASH model still retained 100% sensitivity until the mixture of reads are less than 20% tumor (as observed in several tumor purity and HLA loss of heterozygosity clonality combinations: 20% purity and 100% clonality; 50% purity and 30% clonality; 100% purity and 20% clonality). By contrast, the DASH model retained 100% specificity across all tumor purity levels. For comparison, heatmaps 520 and 525 respectively show specificity and sensitivity levels of the LOHHLA algorithm, across various purity and clonality levels. Similar to above, boxes are shaded differently to indicate various sensitive levels, and grey boxes denotes no data. As shown, the heatmap 515 of the DASH model shows higher sensitivity levels across purity and clonality levels than the heatmap 525 of the LOHHLA algorithm, especially at clonality levels below 80 %.

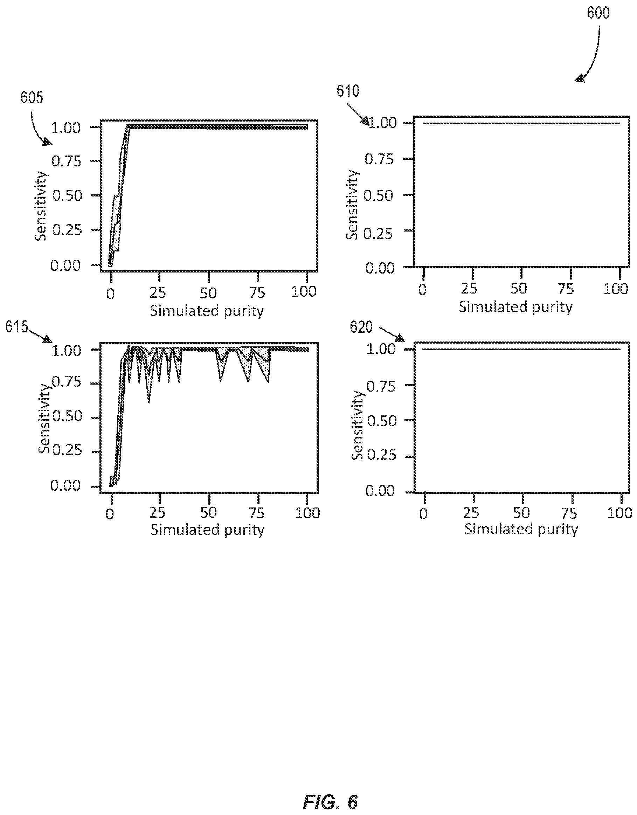

FIG. 6 shows an example of results representing performance levels of the DASH model based on an in silico cell line based limit of detection analysis. The example set of results 600 were based on validation using in silico cell line mixtures and digital PCR. Line plots 605 and 610 show sensitivity and specificity levels of the DASH model. Line plots 615 and 620 show sensitivity and specificity levels of an LOHHLA algorithm. The line plots 605-620 show respective sensitivity and specificity levels at various purity levels with fully clonal tumors. Shaded region in each of the line plots 605-620 denotes 95% confidence. As shown in the line plots 605-620, the DASH model has 100% sensitivity levels across tumor purity above 20% (e.g., the line plot 605), whereas the sensitivity levels corresponding to conventional loss of heterozygosity algorithms are lower at certain tumor purity levels (e.g., the line plot 615). Accordingly, the accuracy and low detection limit of the DASH model can been demonstrated using the in silico cell line mixtures and allele-specific genomic data.

### (2) Validation results for allele-specific genomic validation with digital PCR

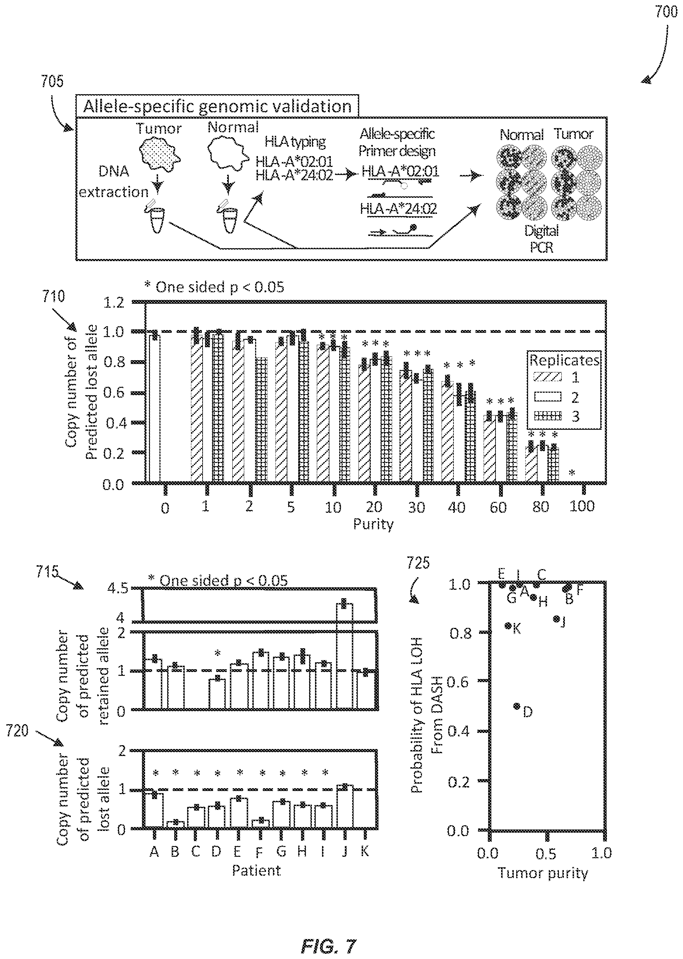

FIG. 7 shows an example of allele-specific genomic technique 700 for validating a machine-learning model trained to detect loss of heterozygosity in HLA alleles, according to some embodiments. After establishing the DASH model's ability to detect HLA loss of heterozygosity in various tumor and normal samples, the prediction accuracy of the DASH model was orthogonally validated using digital PCR. Due to the highly polymorphic nature of HLA alleles, PCR primers and probes were independently designed for each pair of alleles in each subject. In addition to having to target the alleles unique to each subject, the probes were also designed to avoid targets on other subject-specific alleles. As described herein, primers for the predicted retained and lost HLA-C alleles were designed for the cell line interrogated with *in silico* mixtures (NCI-H2009/NCI-BL2009). For both primer targets, the tumor and blood (normal) cell line DNA were mixed to emulate decreasing levels of tumor purity in triplicate and performed digital PCR with both allele-specific designs. The allele primers was multiplexed with RNase P (RPP25) primers as a diploid control. As an illustrative example, the validation technique 700 includes a schematic diagram 705 that illustrates an example of allele-specific genomic validation using paired tumor and adjacent normal fresh frozen samples.

A bar plot 710 shows an allele-specific copy number of the predicted lost allele relative to RNase P, based on the allele-specific genomic validation using digital PCR. The allele-specific number was measured by digital PCR for cell line mixtures of varying tumor purities. To ensure the specificity of the primers, both the predicted lost and predicted retained allele copies normalized by half of the diploid RNase P copies resulted in a one copy in the normal samples.

The bar plot 710 includes a dashed line, which denotes the expected value for no change in copy number. Then, the copy number of each allele in the tumor sample was compared to the normal sample (e.g., using the one-sided Student T test). Asterisks show results indicating whether a statistically significant difference was found based on the comparison with the copy number in the normal sample. One or more copies of the lost allele in the tumor sample were found as tumor purity increased above zero, confirming the allele-specific LOH event. Digital PCR sensitivity 100% for 10% tumor purity and above, confirming the sensitivity and replicability of allele-specific digital PCR as an orthogonal method.

Bar plots 715 and 720 respectively show a ratio between HLA allele digital PCR copy to the multiplexed RNase P digital PCR copy. In the bar plots 715 and 720, cell line data is shown on the left and subject data is shown on the right. In addition, grey bars indicate the ratio in the normal DNA and green bars indicate the ratios in the tumor DNA. The alleles predicted by the DASH model to be retained are shown on the bar plot 715 while the alleles predicted to be deleted are shown on the bar plot 720. The dashed grey lines indicate the expected ratio of 0.5 if there are no copy number alterations. Asterisks indicate samples with p-values less than 0.05 as determined by a one-sided student T test. To ensure the specificity of the primers, it was confirmed that both the predicted lost and predicted retained allele copies normalized by RNase P copies resulted in a 0.5 ratio in the normal sample.

The ratios for 20 of the 22 primers were found to be highly specific and in close proximity to 0.5. However, the predicted retained allele from subject C and the predicted lost allele from subject K were excluded due to low specificity. Then, the ratios of each allele in the tumor sample to the normal sample were compared.

Further, as shown in the asterisks above each bar of the respective bar plots 715 and 720, a significant depletion was found in only one of the nine predicted retained alleles and a significant depletion in eight of the nine predicted lost alleles in the tumor samples. The subject without significant digital PCR depletion in the predicted lost allele (subject J) appears to have a large amplification in the digital PCR of the retained allele. This amplification can be confirmed with a standard copy number variation call in the region surrounding the HLA gene.

A scatter plot 725 shows a distribution of probabilities of HLA loss of heterozygosity returned by the DASH model with their tumor purities. The red region indicates ambiguous calls by the DASH model. The grey vertical line indicates 20% purity. In the scatter plot 725, subject D's predicted retained allele appears to have a slight reduction in the tumor. The significant allelic imbalance in the tumor suggests that this could be due to a subclonal bi-allelic deletion or simply an amplification of the RNase P control. Excluding the call from subject D, 95% of the retained and lost alleles predicted above the 0.8 threshold were identified correctly. Furthermore, several of the samples had low tumor content, confirming the accuracy of the DASH model across variable tumor purities. The subject-specific digital PCR presented here thus represents the first allele-specific genomic HLA loss of heterozygosity validation assay.

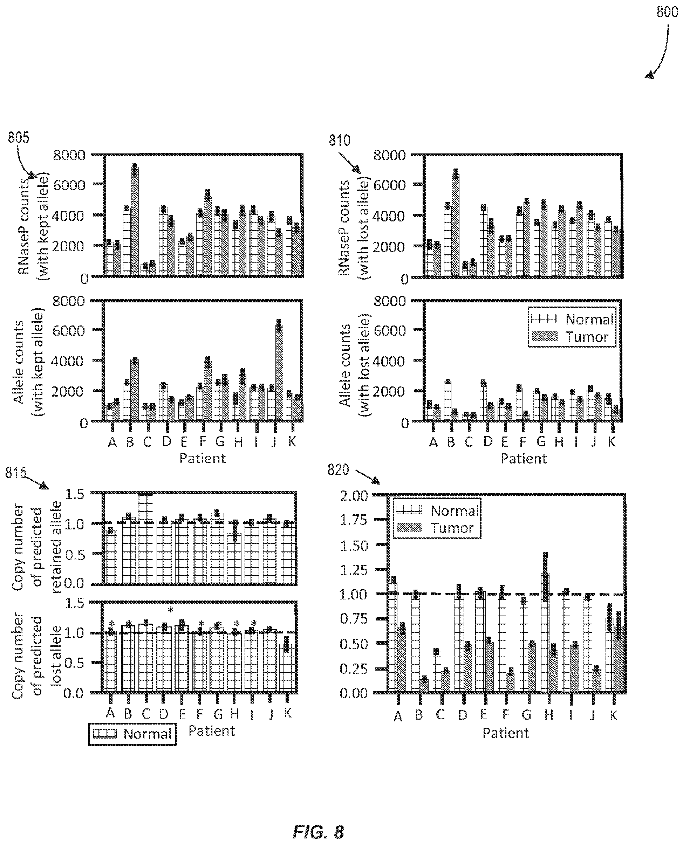

FIG. 8 shows example set of results 800 representing performance levels of the DASH model based on allele-specific genomic validation with digital PCR. Bar plots 805 show digital PCR copies of alleles predicted to be retained, and bar plots 810 show lost digital PCR copies of alleles predicted to be by the DASH model. In both of the bar plots 805 and 810, the multiplexed RNase P was used as the control gene, cell line data is shown on the left portion, and subject data is shown on the right portion. With respect to the bar plots 805 and 810, a set of 11 subjects with corresponding tumor and normal sample pairs were profiled: 4 confirmatory subjects from the training data and data from 7 independent subjects. Primers for the predicted retained and lost alleles were designed, and digital PCR was performed on DNA from normal (adjacent or peripheral blood mononuclear cells) and tumor samples.

Bar plots 815 show specificity of each primer design as measured by a ratio between the allele digital PCR copies and the multiplexed RNase P digital PCR copies in the normal sample. The grey dashed line in each of the bar plots 815 indicates an expected copy number of 1. A bar plot 820 shows a ratio between a predicted lost allele (normalized by RNase P copies) and a predicted retained allele (normalized by RNase P copies) to show allelic imbalance predicted by the DASH model. A dashed grey line of the bar plot 630 shows a ratio of one, which is expected in the normal samples. Deviations below the dash grey line suggest allelic imbalance. Referring to the bar plot 820, the resulting significant allelic imbalance likely caused a lower confidence deletion prediction.

### V. Changes in Peptide Presentation for Tumors with predicted HLA loss of heterozygosity

HLA loss of heterozygosity is hypothesized to reduce the neoantigen load by eliminating surface presentation of neoantigens that would bind to specific HLA alleles. Such hypothesis has been demonstrated with organoids, but it has not been shown in complex subject tumor samples. Thus, in order to provide functional evidence of reduced peptide presentation for alleles that the DASH model predicts are lost, quantitative changes in peptide presentation were measured between adjacent-normal samples without HLA loss of heterozygosity and tumor samples with HLA loss of heterozygosity.

### (a) Overview

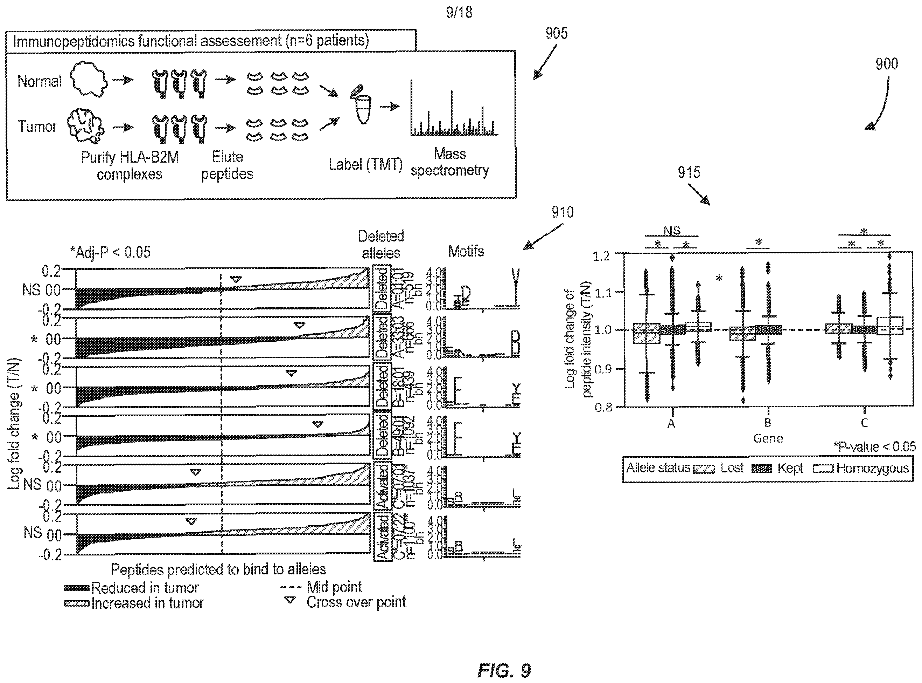

FIG. 9 shows a schematic overview of using immunopeptidomic data for validating a machine-learning model trained to detect loss of heterozygosity in HLA alleles, according to some embodiments. For example, a schematic diagram 905 illustrates a process for applying mass spectrometry to determine which HLA allele was subject to loss of heterozygosity. Specifically, functional immunopeptidomic validation can be performed by: (1) obtaining paired tumor and adjacent normal fresh frozen samples; (2) purifying each of the samples to obtain HLA-Beta-2 microglobulin complexes; and (3) gently eluting peptides from the HLA-Beta-2 microglobulin complexes. The eluted peptides from each sample can be labeled with one or more chemical labels (*e.g.,* a tandem mass tag) and measured using quantitative mass spectrometry.

Waterfall plots 910 show log2 fold change from a normal sample to a tumor sample for peptides binding to each of the alleles in a subject. In the waterfall plots 910, dark color indicates peptides that are less frequent in the tumor while shaded color indicates peptides that are more frequent in the tumor. The dashed grey line represents the mid point of the plot and the triangles indicate the crossover point for each allele. Each waterfall plot of the waterfall plots 910 indicates whether a subject HLA allele has been deleted or retained. The peptides for each allele are visualized as a motif. Statistical significance assessed using a Wilcoxon paired rank sum test. In the waterfall plots 910, three of four deleted alleles (predicted bi-allelic deletions) had significantly fewer predicted binding peptides in the tumor sample than in the adjacent normal sample.

Box plots 915 show log2 fold changes of peptide intensity between lost, kept, and homozygous alleles across HLA-A, HLA-B, and HLA-C alleles. Statistical significance was assessed using a two-sided student T-test. The box plots 915 show that peptides predicted to bind to lost alleles had reduced peptide intensity in tumor samples compared to normal samples for HLA-A and -B alleles.

### (b) Relationship between samples without HLA loss of heterozygosity and samples with predicted HLA loss of heterozygosity

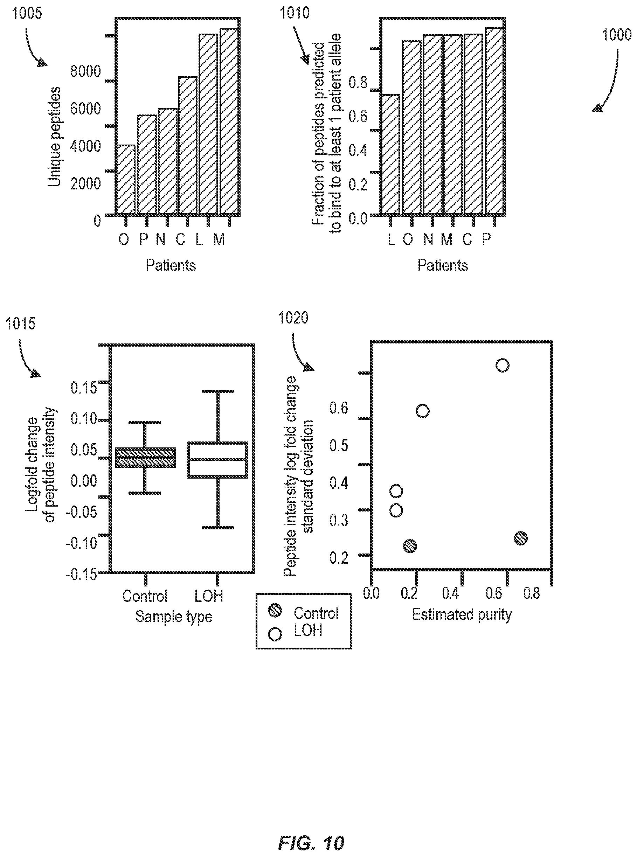

FIG. 10 shows an example of quantitative immunopeptidomics data corresponding to a relationship between control samples without predicted HLA loss of heterozygosity and samples with predicted HLA loss of heterozygosity, according to some embodiments. In this example, quantitative immunopeptidomics data included two control samples without any HLA loss of heterozygosity and four samples with predicted HLA loss of heterozygosity. A bar plot 1005 identifies a number of unique peptides identified in quantitative immunopeptidomics derived from each of the above samples. A bar plot 1010 shows a fraction of peptides in each sample that are predicted to bind to at least one of a corresponding subject's HLA alleles. Across the six samples, the bar plot 805 identified strong peptide yields (median ~5000 unique peptides), and the bar plot 1010 identified a high percentage of observed peptides predicted to bind to at least one of the subject-specific alleles.

A box plot 1015 shows distributions of log2 fold change intensities in samples without HLA loss of heterozygosity (control) and samples with HLA loss of heterozygosity. In the samples without any predicted HLA loss of heterozygosity (M and P samples), minimal differences were found in surface peptide between the tumor and normal samples. For example, the box plot 1015 shows, for the samples without any predicted HLA loss of heterozygosity, an interquartile of peptide log fold changes ranging from -0.010 to 0.013, and the median peptide fold change close to zero for all alleles. In contrast, the samples with predicted HLA loss of heterozygosity (L, C, O and N samples) showed twice as much variability in peptide presentation between the tumor and normal samples, with the interquartile of peptide log fold changes ranging from -0.026 to 0.023.

A scatter plot 1020 shows a relationship between estimated tumor purity and a standard deviation of the log2 fold change of peptide intensities. With respect to the scatter plot 1020, green dots depict samples without HLA loss of heterozygosity and blue dots depict samples with HLA loss of heterozygosity. The deviation of intensities between tumor and normal samples increased as the samples gained in tumor purity, with the highest purity sample showing an average deviation of 0.062 (L, 58% tumor purity).

### (c) Immunopeptidomics data corresponding to samples without predicted HLA loss of heterozygosity

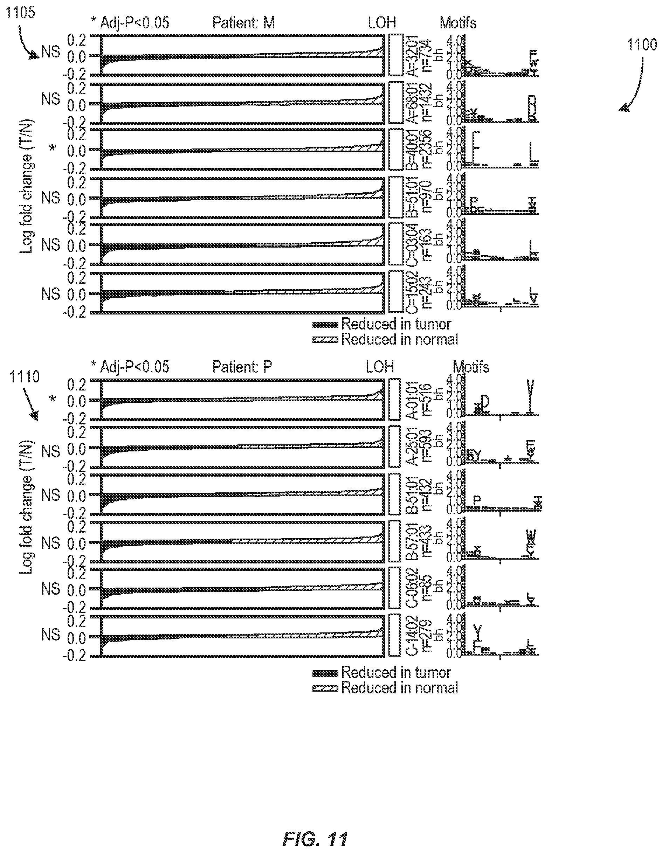

FIG. 11 shows an example of quantitative immunopeptidomics data corresponding to control samples without predicted HLA loss of heterozygosity, according to some embodiments. A waterfall plot 1105 shows immunopeptidomics data corresponding to a first subject without predicted HLA loss of heterozygosity (*e.g*., subject M of FIG. 10). A waterfall plot 1110 shows immunopeptidomics data corresponding to a second subject without predicted HLA loss of heterozygosity (*e.g*., subject P of FIG. 10). The waterfall plots 1105 and 1110 show log2 fold change from a normal sample to a tumor sample for peptides binding to each of the alleles in a particular subject. Dark color indicates peptides that are less frequent in the tumor, while shaded color indicates peptides that are more frequent in the tumor. Each waterfall plot of the waterfall plots 1105 and 1110 indicates that a subject HLA allele has been retained. The peptides for each allele are visualized as a motif. Statistical significance assessed using a Wilcoxon paired rank sum test.

### (d) Immunopeptidomics data corresponding to samples with predicted HLA loss of heterozygosity

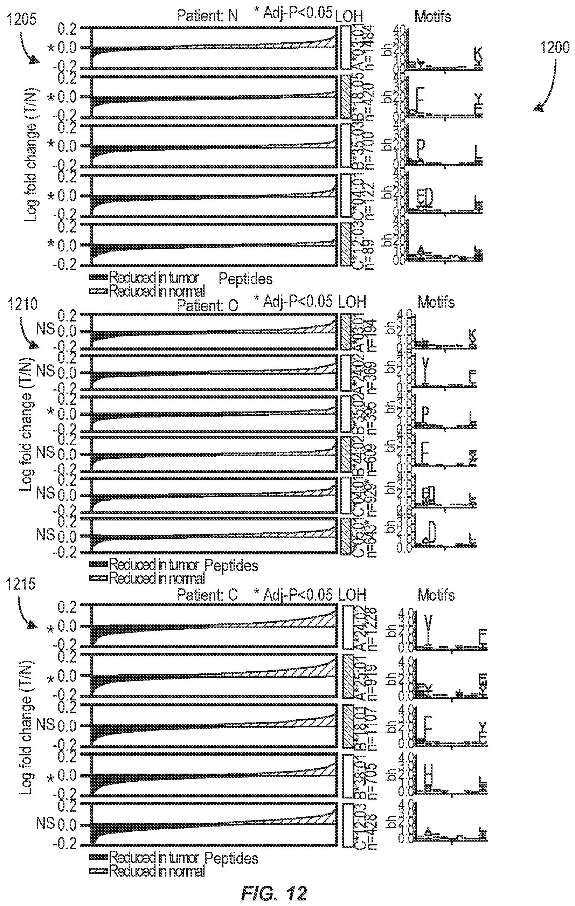

FIG. 12 shows an example of quantitative immunopeptidomics data corresponding to samples with predicted HLA loss of heterozygosity, according to some embodiments. A waterfall plot 1205 shows immunopeptidomics data corresponding to a first subject with predicted HLA loss of heterozygosity (*e.g*., subject N of FIG. 10). A waterfall plot 1210 shows immunopeptidomics data corresponding to a second subject with predicted HLA loss of heterozygosity (*e.g.,* subject O of FIG. 10). A waterfall plot 1215 shows immunopeptidomics data corresponding to a third subject with predicted HLA loss of heterozygosity (*e.g*., subject C of FIG. 10). The waterfall plots 1205-1215 show the log2 fold change from a normal sample to a tumor sample for peptides binding to each of the alleles in a particular subject. Dark color indicates peptides that are less frequent in the tumor, and shaded color indicates peptides that are more frequent in the tumor. The shaded boxes indicate deleted alleles, and white boxes indicate retained alleles. The peptides for each allele are visualized as a motif. Statistical significance assessed using a Wilcoxon paired rank sum test.

Though the three waterfall plots 1205-1215 representing additional low tumor purity samples with predicted HLA loss of heterozygosity had a larger peptide log fold change than the control samples (*e.g.,* the waterfall plots 1105-1110 of FIG. 11), they did not exhibit the same predicted allele-specific peptide depletion (23%, 11% and 11%; C, O and N, respectively) However, the waterfall plot 1205 (23%, C) can be validated as having HLA loss of heterozygosity with digital PCR (subject C represented by the waterfall plot 1215), suggesting that other factors also influence peptide presentation. Although expected variabilities in immunoprecipitation and detection using mass spectrometry limit the sensitivity levels in low purity samples, this is the first functional validation approach of its kind using a subject tumor sample to demonstrate that HLA loss of heterozygosity is associated with changes in peptide presentation.

### VI. Loss of Heterozygosity in HLA Alleles across Tumor Types

HLA loss of heterozygosity prevalence data can demonstrate that a large percentage of subjects are impacted by HLA loss of heterozygosity in several tumor types. Though non-small-cell lung carcinoma is known to have a high incidence of HLA loss of heterozygosity, a large fraction of HLA loss of heterozygosity was identified in other types of cancers, including cervical cancer (44%) and head and neck squamous cell carcinoma (40%). In contrast, only 14% of subjects with HLA loss of heterozygosity were observed in melanoma, which also has a high mutational burden. Further, cervical cancer is strongly associated with human papillomavirus (HPV), which may play a role in the high frequency of HLA loss of heterozygosity. In some instances, subjects lost more than one HLA-allele at a time, potentially having stronger implications on tumor evolution.

To assess the pervasiveness of HLA loss of heterozygosity as a potential immune escape mechanism, the DASH model as applied to 611 tumors across 15 tumor types. A total of 593 subjects from across 14 tumor types were considered for analysis. Each subject had a tumor sample and a normal sample that was sequenced and analyzed. A subset of these samples was used for training the DASH model. The DASH model was applied on each sample to predict the genes (HLA-A, -B and -C) that impacted by HLA loss of heterozygosity. The frequencies of HLA loss of heterozygosity co-occurrence between multiple genes within a single subject were calculated based on a reduced cohort that only contained fully heterozygous subjects.

### (a) HLA loss of heterozygosity predicted across tumor types

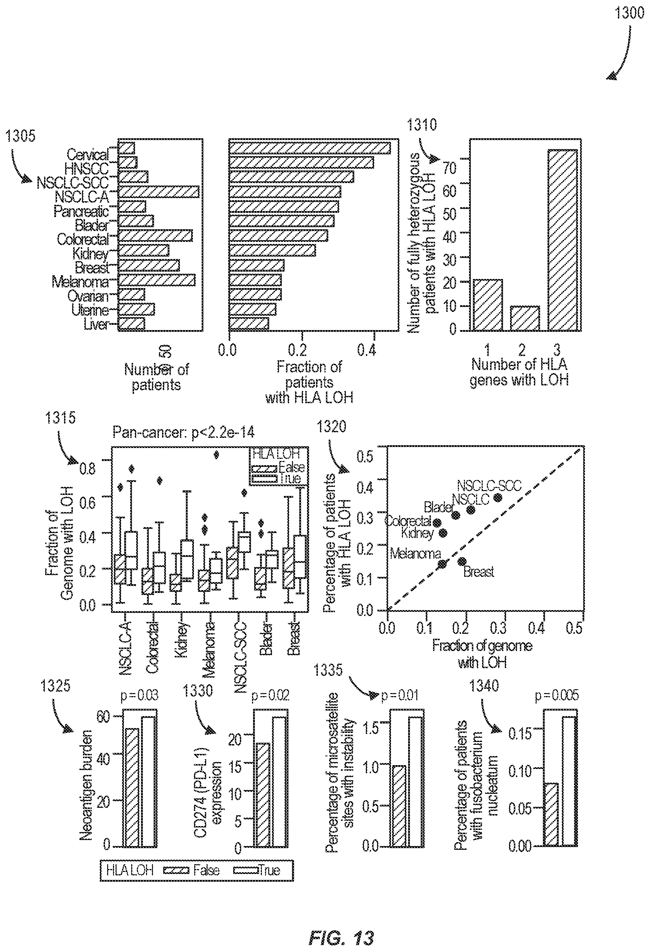

FIG. 13 shows HLA loss of heterozygosity predicted across tumor types, according some embodiments. A bar plot 1305 identifies a number of subjects and the frequency of HLA loss of heterozygosity in each tumor type cohort. In this example, only cohorts with at least 10 subjects are shown. As shown in the bar plot 1305, the fraction of subjects with at least one incidence of HLA loss of heterozygosity ranged from 44% of subjects in cervical cancer to 11% of subjects in liver cancer. For instance, HLA loss of heterozygosity was found in non-small cell lung cancer adenocarcinoma (non-small-cell lung carcinoma-A) at approximately 31%. In another instance, a lower incidence was found in non-small cell lung cancer squamous cell carcinoma (non-small-cell lung carcinoma-SCC) at approximately 34%.

A bar plot 1310 shows the number of subjects with 1, 2 or 3 genes impacted by HLA loss of heterozygosity. In this example, only subjects that are fully heterozygous across HLA-A, -B and -C are shown. In the bar plot 1310, subjects with HLA loss of heterozygosity more frequently lost all three genes (70% of the subjects), compared to losing only one gene or two genes (20% and 10% of subjects, respectively).

A box plot 1315 shows a distribution of the fraction of each genome impacted by HLA loss of heterozygosity. Each tumor type is divided into subjects with HLA loss of heterozygosity and without HLA loss of heterozygosity. Only tumor types with at least 10 subjects impacted by HLA loss of heterozygosity are shown. Statistical analyses are performed with mann whitney U tests and are Bonferroni corrected. Though high frequencies of HLA loss of heterozygosity in specific tumor types are of interest due to impairment of the antigen presentation pathway, the high frequencies alone do not necessitate an evolutionary advantage of the loss of heterozygosity event. As shown in the box plot 1315, it was found that subjects with HLA loss of heterozygosity have significantly higher estimated rates of loss of heterozygosity across their genome, suggesting that some loss of heterozygosity in the HLA region may happen by chance (pan-cancer p<2.2e-14).

To investigate if HLA loss of heterozygosity frequencies across cancer types would occur by chance, the average estimated rate of loss of heterozygosity across the genome was compared with the frequency of HLA loss of heterozygosity in a given tumor type cohort. If loss of heterozygosity was randomly occurring in the HLA region, it would be expected that rate and frequency to be similar. In particular, if a region with an estimated copy number of the B allele is zero, the region can be considered as having HLA loss of heterozygosity. The total number of base pairs impacted by loss of heterozygosity can be totaled for each subject and divided by the total number of base pairs across the exome (3.2 billion) to obtain the fraction of the genome with loss of heterozygosity. Though the fraction may be an underestimate due to limited coverage of genomic regions without genes, the underestimation can be expected to be consistent across subjects.

A scatter plot 1320 shows a relationship between the average fraction of the genome impacted by loss of heterozygosity and the frequency of HLA loss of heterozygosity in each tumor type. The grey dashed line indicates x=y. As shown in the scatter plot 1320, almost all tumor types have a higher frequency of HLA loss of heterozygosity than genome-wide loss of heterozygosity. While this difference is small for some tumor types, it was observed that colorectal cancer, kidney renal clear cell carcinoma, non-small-cell lung carcinoma-A, pancreatic cancer and head and neck squamous cell carcinoma had substantial enrichment of HLA loss of heterozygosity. The data shown in the scatter plot 1320 suggests that HLA loss of heterozygosity may provide a greater evolutionary advantage in these tumor types than others. Alternatively, HLA may be more prone to deletion than the rest of the genome.

Bar plots 1325-1340 show differences of neoantigen expression between subjects without HLA loss of heterozygosity (green) and subjects with HLA loss of heterozygosity (blue), with the assumption that tumors with a greater ability to display neoantigens would be under higher selective pressure to incur HLA loss. The bar plot 1325 shows an average difference of neoantigen burden between two subject categories across various types of cancers. The bar plot 1325 shows a difference between two subject categories. In addition, the remaining bar plots 1330-1340 show difference between the two subject categories. For example, the bar plot 1330 shows a statistically significant difference between two subject categories for CD274 (PD-L1) expression (p = 0.02), the bar plot 1335 shows a statistically significant difference between two subject categories for percentage of microsatellite sites with instability (p = 0.01), and the bar plot 1340 shows a statistically significant difference between two subject categories for percentage of patients with fusobacterium nucleatum, which is an oral bacteria with known colon cancer associations (p = 0.005). Accordingly, the box plots 1325-1340 show that there could be a possibility that tumors with a greater ability to display neoantigens can cause HLA loss of heterozygosity.

### (b) Antigen presentation and loss of heterozygosity

Since HLA loss of heterozygosity impacts the ability of a tumor cell to present antigen on the cell surface for recognition by the immune system, it was hypothesized that tumors with a greater ability to display neoantigens would be under higher selective pressure to incur HLA loss. Considering subjects with high HLA evolutionary diversity are able to present a larger immunopeptidome and respond better to checkpoint inhibitors, experiments were conducted to determine if such subjects are more susceptible to HLA loss.

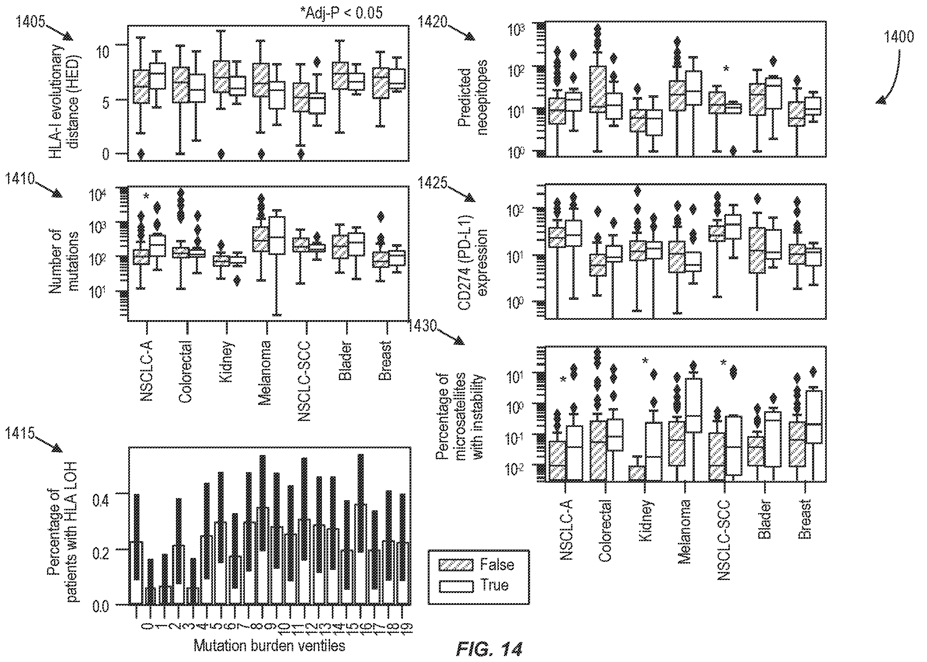

FIG. 14 shows a set of experimental results 1400 that show relationship between HLA loss of heterozygosity and antigen presentation across various tumor types, according to some embodiments. A boxplot 1405 shows a distribution of HLA-I evolutionary divergence across subjects with and without HLA loss of heterozygosity. Only tumor types with at least 8 subjects impacted by HLA loss of heterozygosity are shown. Statistical analyses are performed with mann whitney U tests and are Bonferroni corrected. The germline HLA-I evolutionary divergence score was calculated from the HLA alleles of each individual subject. The HLA-I evolutionary divergence score is intended to capture allelic sequence diversity for a subject's HLA alleles, with a low score indicating low diversity and a high score indicating high diversity. The boxplot 1405 shows that there appears to be no substantial correlation between HLA evolutionary diversity and HLA loss of heterozygosity.

Further, a boxplot 1410 shows a distribution corresponding to a number of mutations (*e.g*., single-nucleotide variant, indel and fusion) across subjects with and without HLA loss of heterozygosity. Only tumor types with at least 8 subjects impacted by HLA loss of heterozygosity are shown. Statistical analyses are performed with mann whitney U tests and are Bonferroni corrected. Mutational burdens-a number of mutations-were identified using tumor-specific genomic events of at least 5% allelic fraction that were verified using transcriptomic data. All potential neoepitopes (8-,9-,10- and 11-mers) were created for each mutation and tested for presentation. If any 8-,9-,10- or 11-mers containing the mutation were predicted to bind to any of the subject-specific alleles, they are considered putative neoepitopes. The boxplot 1410 shows high mutation rates and neoantigen burdens can present pressure for cells to lose HLA. A boxplot 1415 shows percentage of patients with HLA LOH in each ventile of mutation burden pan-cancer. Both of the boxplots 1410 and 1415 show "goldilocks effect" for mutation burdens, in which diseases with the lowest tumor mutational burden and highest tumor mutational burden exhibited the lowest prevalence of HLA loss of heterozygosity, whereas tumors in between exhibited the highest prevalence of HLA loss of heterozygosity.

A boxplot 1420 shows a distribution of predicted neoepitopes across subjects with and without HLA loss of heterozygosity across each of various types of cancers. Statistical analyses are performed with mann whitney U tests and are Bonferroni corrected. The boxplot 1420 shows pan-cancer evidence for a correlation with neoantigen burden (p = 0.03). Further, a boxplot 1425 shows correlations between HLA loss of heterozygosity and CD274 expression (PD-L1) across each of various types of cancers (p=0.02), and a boxplot 1430 shows correlations between HLA loss of heterozygosity and microsatellite instability (MSI) status across each of various types of cancers (p= 0.01). It was found that more neoantigens are predicted to bind to lost HLA alleles than their homologous counterparts (Wilcoxon rank sum test, p=0.01), thereby suggesting that HLA loss of heterozygosity contributes to the selective exposure of antigen to the immune system.

### VII. Tumor Cell Responses to immune checkpoint blockade therapies

The allele-specific neoantigen composition changes detected in the head and neck squamous cell carcinoma cohort suggest that HLA loss of heterozygosity is altering tumor evolution in response to immune checkpoint blockade therapies. This observation corroborates that HLA sequence variability is a component of effective immune checkpoint blockade response by tumor cells. Though larger cohorts with detailed response data are needed to confirm the impact of HLA loss of heterozygosity on subject response and survival, the examples shown below suggest that accurate detection of HLA loss of heterozygosity will be a factor for checkpoint immunotherapy and cancer vaccine target selection.

Although HLA loss of heterozygosity appears to apply limited evolutionary pressure during tumor growth, immune checkpoint inhibitors serve to increase immune pressure. Thus, the impact of HLA loss of heterozygosity in response to immunotherapy was investigated. Since HLA loss of heterozygosity severely reduces the immunopeptidome by eliminating several HLA alleles, it was reasoned that HLA loss of heterozygosity should impair response to immunotherapy through reduced MHC presentation.

To identify HLA loss of heterozygosity in response to immunotherapies, an experiment was conducted to identify HLA loss of heterozygosity on a cohort of seven head and neck squamous cell carcinoma subjects who received a single dose of PD-1 inhibitor (nivolumab). The pre- and post-treatment tumor biopsies corresponding to the subjects were sequenced. The interaction between germline variability in HLA sequence and pretreatment somatic alterations to antigen presentation machinery was identified. Using the DASH model, four subjects with HLA loss of heterozygosity were found. Moreover, Beta-2 microglobulin loss of heterozygosity in one subject and somatic mutations in HLA alleles of two other subjects were discovered. The three subjects with the most germline HLA sequence diversity all suffered from HLA loss of heterozygosity, with the subject with the highest diversity also having Beta-2 microglobulin loss of heterozygosity.

### (a) Cohort population

Pre- and post-intervention matched normal, tumor and plasma samples were collected from a cohort of 7 subjects with head and neck squamous cell carcinoma . Following baseline sample collection all subjects received a single dose of nivolumab, followed by definitive resection of the primary tumor mass approximately one month later when feasible, or a second biopsy where resection was impractical. Due to the resection protocol, RECIST criteria was not used to evaluate response in resected subjects. Solid tumor and matched normal samples were profiled.

### (b) Neoantigen expansion to immunotherapy response

For each subject with pre- and post-treatment samples, the DASH model can be used to predict occurrence of HLA loss of heterozygosity. In addition, an HLA evolutionary diversity score can be determined for detecting HLA somatic mutation and Beta-2 microglobulin loss of heterozygosity. Potential epitopes for each mutation detected pre- or post-treatment can be predicted to detect binding with all subject-specific alleles, as described above. Neoepitopes can be identified from mutations that are observed post-treatment but are not observed pre-treatment. In some instances, a neoepitope is predicted to bind to multiple HLA alleles. Some neoepitopes bound to homozygous alleles can be excluded. A paired Wilcoxon Rank Sum Test can be performed to assess the statistical significance of the number of novel neoepitopes predicted to bind to the lost HLA-A/-B alleles and their retained homologous allele.

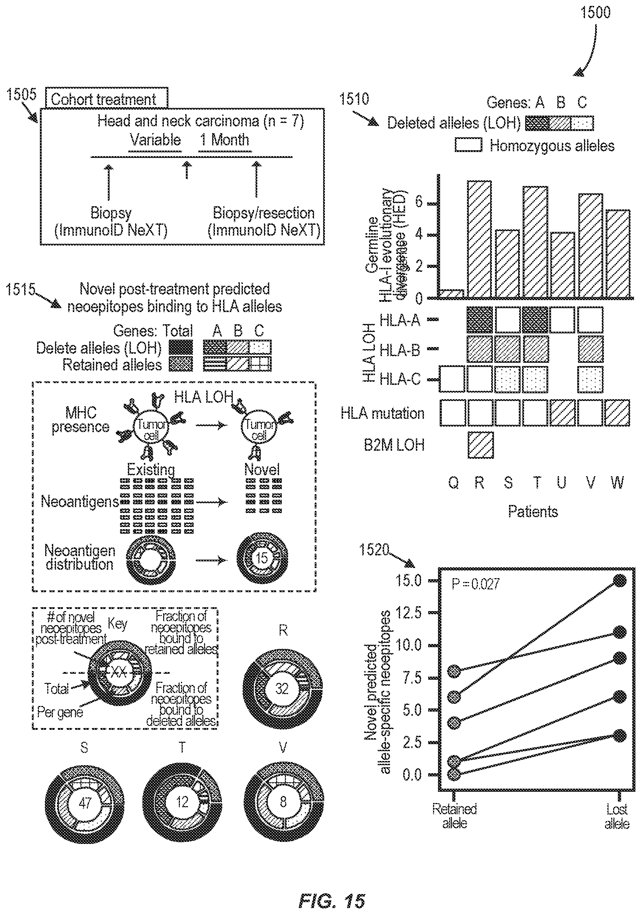

FIG. 15 shows experiment data that identify neoantigen expansion in response to immune checkpoint inhibitor therapy on head and neck squamous cell carcinoma subjects, according to some embodiments. It was expected that clones with neoantigens presentable by the lost alleles would expand in response to the immunotherapy. Further, it was expected that clones with neoantigens presentable by the intact alleles contract in response to the immunotherapy. The experiment data correspond to an analysis of a head and neck squamous cell carcinoma cohort due to its high prevalence of HLA loss of heterozygosity (44% of subjects) and evidence of positive selection. Further, one of the treatment regimens applied for this cohort included a PD-1 inhibitor (pembrolizumab), which is a first line treatment for PD-L1 positive head and neck squamous cell carcinoma subjects. Previous data suggest that only a small fraction of subjects show strong response to PD-1 inhibitor. Moreover, PD-1 inhibitor treatments using pembrolizumab alone resulted in adverse events that led to death in 8% of the corresponding subjects. Thus, an understanding the mechanisms that lead to response can be used to increase subject survival.

In FIG. 15, subject data 1505 provides that, in the experiment data, seven subjects (n=7) with head and neck squamous cell carcinoma were biopsied before being treated with nivolumab. Approximately one month later, each subject either had another biopsy or a resection. All tumor samples, pre- and post-treatment, were profiled to identifies various types of immune-related information, including tumor mutational burden, HLA typing, etc.

Bars in a bar graph 1510 indicate an HLA-I Evolutionary Divergence score for each subject in the cohort population identified in Section VII(a) herein. For HLA genes, shaded boxes indicate somatic loss of heterozygosity and shaded boxes for rows "HLA mutation" and "B2M LOH" indicate a mutation in an HLA gene or loss of heterozygosity in the Beta-2 microglobulin gene, respectively. Homozygous alleles and alleles with very few differences are noted with grey squares.

Circle plots 1515 indicate a ratio of novel post-treatment neoantigens predicted to bind to each of a subject's HLA alleles. Portions of each circle plot is shaded differently to identify whether HLA alleles are deleted or retained. The outer circle shows the ratio of neoepitopes predicted to bind to all lost and retained alleles, and the inner circle shows the breakdown by specific allele. The value inside the circle represents the number of novel neoepitopes predicted post treatment (multi-counted if predicted to be presented by multiple alleles). For the circle plots 1515, neoepitopes presented by homozygous alleles were excluded.

As shown in the circle plots 1515, in each subject with HLA loss of heterozygosity, it was found that more new post-treatment neoantigens were predicted to bind to deleted HLA alleles than to the retained HLA alleles for the corresponding subject.

A scatter-line plot 1520 shows a paired relationship between the count of novel, post-treatment allele-specific predicted neoepitopes for retained and deleted HLA alleles. Only HLA-A and -B alleles are shown in the scatter-line plot 1520. Statistical significance is assessed using a Wilcoxon paired rank test. Since sequence diversity in HLA-A and -B alleles alone may have an impact on response to immunotherapy, the number of novel post-treatment neoantigens predicted to bind to HLA-A and-B alleles were compared to their homologous counterparts and found a statistically significant difference across the cohort (p=0.027, Wilcoxon signed-rank). This consistent shift in neoantigen composition suggests that HLA loss of heterozygosity acts as an evolutionary force in resistance to response during immunotherapy.

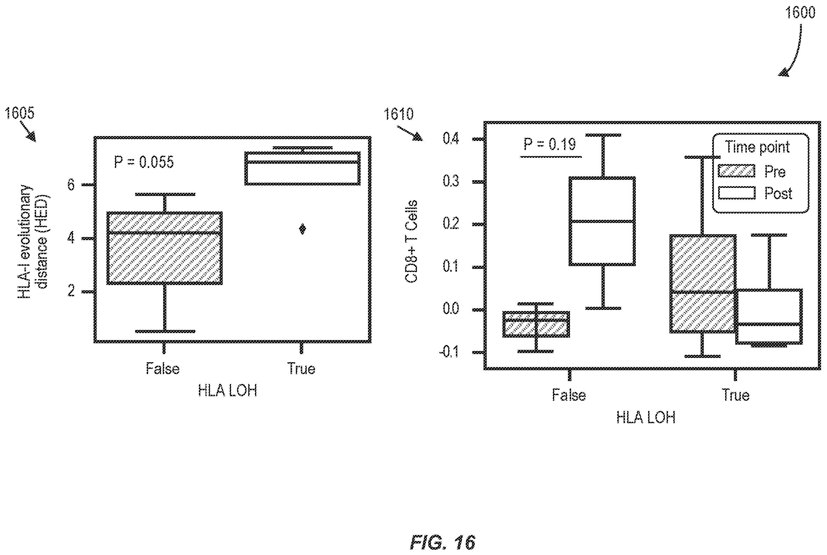

FIG. 16 shows additional experiment data corresponding to HLA loss of heterozygosity on tumors treated with immunotherapy, according to some embodiments. Box plots 1605 shows a distribution of HLA-I Evolutionary Distance scores for subjects with and without HLA loss of heterozygosity. Statistical significance is performed using a Mann-Whitney U test. In the box plots 1605, the subject with the least germline HLA sequence diversity (HLA-I Evolutionary Divergence < 1) had no somatic alterations to the antigen presentation machinery.

Box plots 1610 shows a difference in estimated tumor infiltrating CD8+ T cell quantification pre- and post-treatment for subjects with and without HLA loss of heterozygosity. Statistical significance is performed using a Mann-Whitney U test. In the box plots 1610, a trend was observed toward increased CD8+ T cells after treatment for subjects without HLA loss of heterozygosity. In contrast, the same trend was not observed in subjects with HLA loss of heterozygosity. The difference between samples shown in the box plots 1610 suggest that a decrease in diversity of neoantigens may reduce immune infiltration.

### VIII. Process for Predicting Loss of Heterozygosity in HLA Alleles Using Machine-Learning Models

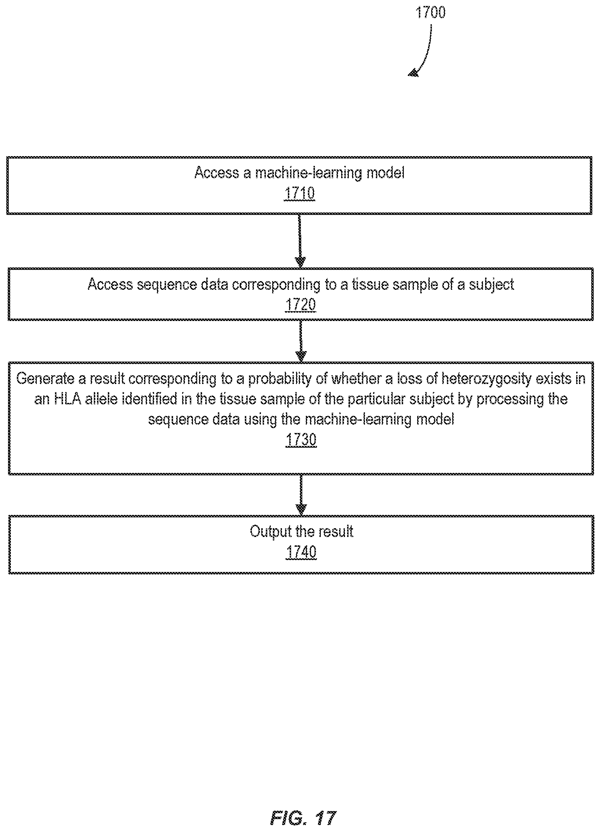

FIG. 17 includes a flowchart 1700 illustrating an example of a method of predicting loss of heterozygosity in HLA alleles, according to certain some embodiments. Operations described in flowchart 1700 may be performed by, for example, a computer system implementing a trained machine-learning model, such as the DASH model. Although flowchart 1700 may describe the operations as a sequential process, in various embodiments, many of the operations may be performed in parallel or concurrently. In addition, the order of the operations may be rearranged. An operation may have additional steps not shown in the figure. Furthermore, some embodiments of the method may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium.

At operation 1710, a computer system accesses a machine-learning model. The machine-learning model can be trained using a training data set that included, for a subject of a set of subjects: (1) allele-specific features; (2) subject-specific features; and (3) whole-exome features. The allele-specific features can include, for a genomic region of an HLA allele: an adjusted B allele frequency that represents a ratio between a first B allele frequency of heterozygous alleles in the tumor sample that correspond to the genomic region and a second B allele frequency of heterozygous alleles in the genomic region and associated with one or more control samples; and a ratio between a first allele-specific coverage of the tumor sample that corresponds to the genomic region and a second allele-specific coverage of the one or more control samples that corresponds to the genomic region. In some instances, the allele-specific features correspond to a genomic region of an HLA allele that was identified as having a somatic mutation.

The subject-specific features can include an estimated tumor purity value and an estimated tumor ploidy value corresponding to a tumor sample of the subject. Tumor purity, as used herein, refers to as a ratio of tumor cells to total cells in the sample. Tumor ploidy, as used herein, refers to an average copy number of the entire tumor genome. The whole-exome features can include, for the HLA allele, an indication of whether at least part of a flanking genomic region surrounding the HLA allele has been deleted. Example embodiments for training the machine-learning model can be found in Section III of the present disclosure.

Performance of the trained machine-learning model can be evaluated by using one or more validation techniques. For example, the machine-learning model can be validated using *in silico* cell line mixtures, subject-specific primers and probes generated using digital PCR, and/or immunopeptidomics data corresponding to the training data set. Example embodiments for validating the machine-learning model can be found in Section IV of the present disclosure.

At operation 1720, the computer system accesses sequence data corresponding to a biological sample of a particular subject. The biological sample can be a tissue sample of the particular subject that may include DNA derived from cancer cells. In some instances, the sequence data is derived from the biological sample and a reference sample that does not include cancer cells. The biological sample can include cell-free DNA, some of which can have originated from healthy cells and some from tumor cells. The sequence data can be profiled to identify various characteristics corresponding to the biological sample. For example, the characteristics may include comprehensive tumor mutation information, gene expression quantification, neoantigen characterization, HLA alleles (types and mutations), and tumor microenvironment profiling.

In some instances, the sequence data is generated by using whole genome sequencing or whole exome sequencing on the biological sample to generate a plurality of sequence reads. In some instances, HLA genotyping is performed on the plurality of sequence reads to identify one or more HLA alleles that correspond to the sequence data. Reference sequences corresponding to the identified HLA alleles can be retrieved, and the sequence reads can be aligned to the retrieved reference sequences. After alignment, allele-specific coverage for each genomic region can be determined for the identified HLA alleles corresponding to the sequence data. In some instances, the aligned sequence data can be analyzed to identify allele-specific copy number alterations from the particular HLA allele-type. Additionally or alternatively, the sequencing data can be analyzed to estimate tumor purity (alternatively referred to as tumor cellularity) and tumor ploidy. Example embodiments for generating the sequence data can be found in at least Section I of the present disclosure.

At operation 1730, the computer system generates a result corresponding to a probability of whether a loss of heterozygosity exists in an HLA allele identified in the tissue sample of the particular subject by processing the sequence data using the machine-learning model. The machine-learning model (e.g., the DASH model) uses the allele-specific data for each of the identified HLA alleles as an input to generate the result. Other types of information corresponding to the identified HLA alleles (e.g., an indication of whether at least part of a flanking genomic region surrounding the HLA allele has been deleted) can be used as additional input to the trained machine-learning model. The machine-learning model can include one or more gradient boosting algorithms to process the above features of the sequence data to generate the result.

In some instances, the result is used to predict a decrease in efficacy of an immune checkpoint blockade therapy being administered to the particular subject. The result can be used to predict a particular type of cancer associated with the subject, as the tumor samples with predicted HLA loss of heterozygosity can be identified as having the particular type of cancer based on their corresponding changes in peptide presentation.

At operation 1740, the computer system outputs the result. Process 1700 terminates thereafter.

### IX. Computing Environment

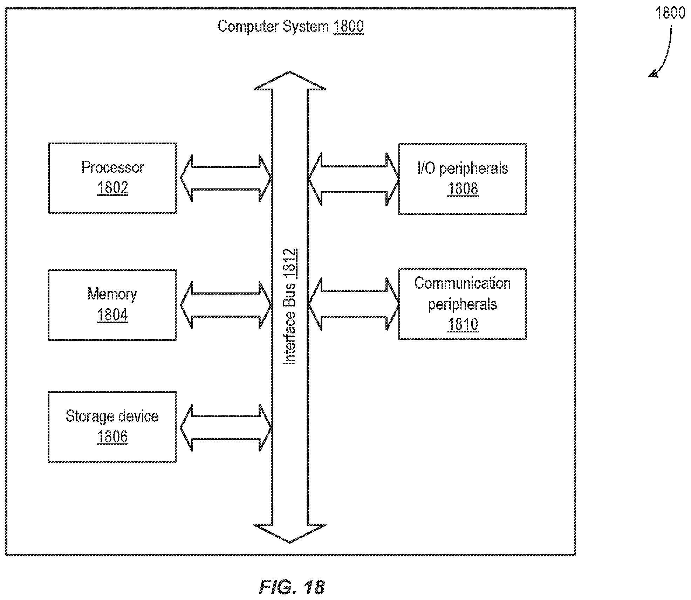

**FIG. 18** illustrates an example of a computer system 1800 for implementing some of some embodiments disclosed herein. The computer system 1800 may include a distributed architecture, where some of the components (*e.g*., memory and processor) are part of an end user device and some other similar components (*e.g*., memory and processor) are part of a computer server. In some instances, the computer system 1800 is a computer system that predicts loss of heterozygosity in HLA alleles using a machine-learning model, which includes at least a processor 1802, a memory 1804, a storage device 1806, input/output (I/O) peripherals 1808, communication peripherals 1810, and an interface bus 1812. The interface bus 1812 is configured to communicate, transmit, and transfer data, controls, and commands among the various components of computer system 1800. The processor 1802 may include one or more processing units, such as CPUs, GPUs, TPUs, systolic arrays, or SIMD processors. Memory 1804 and storage device 1806 include computer-readable storage media, such as RAM, ROM, electrically erasable programmable read-only memory (EEPROM), hard drives, CD-ROMs, optical storage devices, magnetic storage devices, electronic non-volatile computer storage, for example, Flash^{®} memory, and other tangible storage media. Any of such computer-readable storage media can be configured to store instructions or program codes embodying aspects of the disclosure. Memory 1804 and storage device 1806 also include computer-readable signal media.

A computer-readable signal medium includes a propagated data signal with computer-readable program code embodied therein. Such a propagated signal takes any of a variety of forms including, but not limited to, electromagnetic, optical, or any combination thereof. A computer-readable signal medium includes any computer-readable medium that is not a computer-readable storage medium and that can communicate, propagate, or transport a program for use in connection with computer system 1800.

Further, the memory 1804 includes an operating system, programs, and applications. The processor 1802 is configured to execute the stored instructions and includes, for example, a logical processing unit, a microprocessor, a digital signal processor, and other processors. For example, the computing system 1800 can execute instructions (e.g., program code) that configure the processor 1802 to perform one or more of the operations described herein. The program code includes, for example, code implementing the training the DASH model, using the DASH model, accessing the sequence data, and/or any other suitable applications that perform one or more operations described herein. The instructions could include processor-specific instructions generated by a compiler or an interpreter from code written in any suitable computer-programming language, including, for example, C, C++, C#, Visual Basic, Java, Python, Perl, JavaScript, and ActionScript.

The program code can be stored in the memory 1804 or any suitable computer-readable medium and can be executed by the processor 1802 or any other suitable processor. In some embodiments, all modules in the computer system for predicting loss of heterozygosity in HLA alleles are stored in the memory 1804. In additional or alternative embodiments, one or more of these modules from the above computer system are stored in different memory devices of different computing systems.

The memory 1804 and/or the processor 1802 can be virtualized and can be hosted within another computing system of, for example, a cloud network or a data center. I/O peripherals 1808 include user interfaces, such as a keyboard, screen (*e.g.,* a touch screen), microphone, speaker, other input/output devices, and computing components, such as graphical processing units, serial ports, parallel ports, universal serial buses, and other input/output peripherals. The I/O peripherals 1808 are connected to the processor 1802 through any of the ports coupled to the interface bus 1812. The communication peripherals 1810 are configured to facilitate communication between the computer system 1800 and other computing devices over a communications network and include, for example, a network interface controller, modem, wireless and wired interface cards, antenna, and other communication peripherals. For example, the computing system 1800 is able to communicate with one or more other computing devices (e.g., a computing device that is used for training and validating the DASH model, a computing device that displays outputs generated by the DASH model) via a data network using the a network interface device of the communication peripherals 1810.

While the present subject matter has been described in detail with respect to specific embodiments thereof, it will be appreciated that those skilled in the art, upon attaining an understanding of the foregoing may readily produce alterations to, variations of, and equivalents to such embodiments. Accordingly, it should be understood that the present disclosure has been presented for purposes of example rather than limitation, and does not preclude inclusion of such modifications, variations, and/or additions to the present subject matter as would be readily apparent to one of ordinary skill in the art.

Unless specifically stated otherwise, it is appreciated that throughout this specification discussions utilizing terms such as "processing," "computing," "calculating," "determining," and "identifying" or the like refer to actions or processes of a computing device, such as one or more computers or a similar electronic computing device or devices, that manipulate or transform data represented as physical electronic or magnetic quantities within memories, registers, or other information storage devices, transmission devices, or display devices of the computing platform.

The system or systems discussed herein are not limited to any particular hardware architecture or configuration. A computing device can include any suitable arrangement of components that provide a result conditioned on one or more inputs. Suitable computing devices include multipurpose microprocessor-based computing systems accessing stored software that programs or configures the computing system from a general purpose computing apparatus to a specialized computing apparatus implementing one or more embodiments of the present subject matter. Any suitable programming, scripting, or other type of language or combinations of languages may be used to implement the teachings contained herein in software to be used in programming or configuring a computing device.

Certain embodiments of the methods disclosed herein may be performed in the operation of such computing devices. The order of the blocks presented in the examples above can be varied-for example, blocks can be re-ordered, combined, and/or broken into sub-blocks. Certain blocks or processes can be performed in parallel.

Conditional language used herein, such as, among others, "can," "could," "might," "may," "*e.g.*," and the like, unless specifically stated otherwise, or otherwise understood within the context as used, is generally intended to convey that certain examples include, while other examples do not include, certain features, elements, and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. The use of "adapted to" or "configured to" herein is meant as open and inclusive language that does not foreclose devices adapted to or configured to perform additional tasks or steps. Additionally, the use of "based on" is meant to be open and inclusive, in that a process, step, calculation, or other action "based on" one or more recited conditions or values may, in practice, be based on additional conditions or values beyond those recited. Similarly, the use of "based at least in part on" is meant to be open and inclusive, in that a process, step, calculation, or other action "based at least in part on" one or more recited conditions or values may, in practice, be based on additional conditions or values beyond those recited. Headings, lists, and numbering included herein are for ease of explanation only and are not meant to be limiting.

The various features and processes described above may be used independently of one another, or may be combined in various ways. All possible combinations and sub-combinations are intended to fall within the scope of the present disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed examples. Similarly, the example systems and components described herein may be configured differently than described. For example, elements may be added to, removed from, or rearranged compared to the disclosed examples.

## Claims

1. A computer-implemented method comprising:
accessing a machine-learning model, wherein the machine-learning model was trained using a training data set that included one or more sets of training features corresponding to a human leukocyte antigen (HLA) allele identified in a tumor sample corresponding to a subject of a set of subjects:
wherein the training features include, for a genomic region of the HLA allele:
(1) an adjusted B allele frequency that represents a ratio between a first B allele frequency of heterozygous alleles in the tumor sample that correspond to the genomic region and a second B allele frequency of heterozygous alleles in the genomic region and associated with one or more control samples;
(2) a ratio between a first allele-specific coverage of the tumor sample that corresponds to the genomic region and a second allele-specific coverage of the one or more control samples that corresponds to the genomic region; and
(3) an indication of whether at least part of a flanking genomic region surrounding the HLA allele has been deleted;
receiving sequence data corresponding to a biological sample of a particular subject;
generating a result corresponding to a probability of whether a loss of heterozygosity exists in an HLA allele identified in the biological sample of the particular subject by processing the sequence data using the machine-learning model; and
outputting the result;
further comprising predicting, based on the result, one or more neoantigens that correspond to the HLA allele identified in the biological sample of the particular subject.

2. The method of claim 1, wherein the sequence data is whole exome sequencing data.

3. The method of any one of claims 1-2, wherein the sequence data is whole genome sequencing data.

4. The method of any one of claim 1-3, wherein the machine-learning model is trained using the training data set that further included a tumor purity value and a tumor ploidy value corresponding to the subject.

5. The method of any one of claim 1-4, wherein the training data set is generated using a reference sequence of the HLA allele corresponding to the subject.

6. The method of any one of claim 1-5, wherein the machine-learning model includes one or more trained gradient boosting algorithms.

7. The method of any one of claim 1-6, further comprising predicting, based on the result, a decrease in efficacy of an immune checkpoint blockade therapy being administered to the particular subject.

8. The method of any one of claim 1-7, wherein the biological sample of the particular subject includes one or more cancer cells.

9. The method of any one of claim 1-8, wherein processing the sequence data using the machine-learning model includes determining allele-specific data for an HLA allele identified from the sequence data.

10. The method of claim 9, wherein the HLA allele is identified from the sequence data by applying HLA genotyping to the sequence data.

11. A system comprising:
one or more data processors; and
a non-transitory computer readable storage medium containing instructions which, when executed on the one or more data processors, cause the one or more data processors to perform part or all of a method according to any of claims 1 to 10.

12. A computer-program product tangibly embodied in a non-transitory machine-readable storage medium, including instructions configured to cause one or more data processors to perform part or all of a method according to any of claims 1 to 10.

## Patentansprüche

1. Computerimplementiertes Verfahren, umfassend:
Zugreifen auf ein Maschinenlernmodell, wobei das Maschinenlernmodell unter Verwendung eines Trainingsdatensatzes trainiert wurde, der einen oder mehrere Sätze von Trainingsmerkmalen beinhaltete, die einem humanen Leukozyten-Antigen(HLA-)Allel entsprechen, das in einer Tumorprobe identifiziert ist, die einem Subjekt aus einem Satz von Subjekten entspricht;
wobei die Trainingsmerkmale für eine genomische Region des HLA-Allels Folgendes beinhalten:
(1) eine angepasste B-Allel-Frequenz, die ein Verhältnis zwischen einer ersten B-Allel-Frequenz von heterozygoten Allelen in der Tumorprobe, die der genomischen Region entsprechen, und einer zweiten B-Allel-Frequenz von heterozygoten Allelen in der genomischen Region und assoziiert mit einer oder mehreren Kontrollproben darstellt;
(2) ein Verhältnis zwischen einer ersten allelspezifischen Abdeckung der Tumorprobe, die der genomischen Region entspricht, und einer zweiten allelspezifischen Abdeckung der einen oder der mehreren Kontrollproben, die der genomischen Region entspricht; und
(3) eine Angabe, ob zumindest Teil einer flankierenden genomischen Region, die das HLA-Allel umgibt, deletiert worden ist;
Empfangen von Sequenzdaten, die einer biologischen Probe eines bestimmten Subjekts entsprechen;
Erzeugen eines Ergebnisses, das einer Wahrscheinlichkeit entspricht, ob ein Verlust an Heterozygotie in einem HLA-Allel existiert, das in der biologischen Probe des bestimmten Subjekts identifiziert ist, durch Verarbeiten der Sequenzdaten unter Verwendung des Maschinenlernmodells; und
Ausgeben des Ergebnisses;
ferner umfassend Vorhersagen, basierend auf dem Ergebnis, von einem oder mehreren Neoantigenen, die dem HLA-Allel entsprechen, das in der biologischen Probe des bestimmten Subjekts identifiziert ist.

2. Verfahren nach Anspruch 1, wobei die Sequenzdaten Ganz-Exom-Sequenzierungsdaten sind.

3. Verfahren nach einem der Ansprüche 1-2, wobei die Sequenzdaten Ganz-Genom-Sequenzierungsdaten sind.

4. Verfahren nach einem von Anspruch 1-3, wobei das Maschinenlernmodell unter Verwendung des Trainingsdatensatzes trainiert wird, der ferner einen Tumorreinheitswert und einen Tumorploidiewert, der dem Subjekt entspricht, beinhaltete.

5. Verfahren nach einem von Anspruch 1-4, wobei der Trainingsdatensatz unter Verwendung einer Referenzsequenz des HLA-Allels, das dem Subjekt entspricht, erzeugt wird.

6. Verfahren nach einem von Anspruch 1-5, wobei das Maschinenlernmodell einen oder mehrere trainierte Gradientenverstärkungsalgorithmen beinhaltet.

7. Verfahren nach einem von Anspruch 1-6, ferner umfassend Vorhersagen, basierend auf dem Ergebnis, einer Abnahme der Wirksamkeit einer Immun-Checkpoint-Blockade-Therapie, die dem bestimmten Subjekt verabreicht wird.

8. Verfahren nach einem von Anspruch 1-7, wobei die biologische Probe des bestimmten Subjekts eine oder mehrere Krebszellen beinhaltet.

9. Verfahren nach einem von Anspruch 1-8, wobei das Verarbeiten der Sequenzdaten unter Verwendung des Maschinenlernmodells Bestimmen von allelspezifischen Daten für ein HLA-Allel, das aus den Sequenzdaten identifiziert ist, beinhaltet.

10. Verfahren nach Anspruch 9, wobei das HLA-Allel aus den Sequenzdaten durch Anwenden von HLA-Genotypisierung auf die Sequenzdaten identifiziert wird.

11. System, umfassend:
einen oder mehrere Datenprozessoren; und
ein nichtflüchtiges computerlesbares Speichermedium, das Anweisungen enthält, die, wenn auf dem einen oder den mehreren Datenprozessoren ausgeführt, den einen oder die mehreren Datenprozessoren veranlassen, Teil oder alles eines Verfahrens nach einem der Ansprüche 1 bis 10 durchzuführen.

12. Computerprogrammprodukt, das greifbar in einem nichtflüchtigen maschinenlesbaren Speichermedium verkörpert ist, das Anweisungen beinhaltet, die dazu konfiguriert sind, einen oder mehrere Datenprozessoren zu veranlassen, Teil oder alles eines Verfahrens nach einem der Ansprüche 1 bis 10 durchzuführen.

## Revendications

1. Procédé mis en œuvre par ordinateur comprenant :
l'accès à un modèle d'apprentissage automatique, ledit modèle d'apprentissage automatique ayant été formé au moyen d'un ensemble de données de formation qui comprenait un ou plusieurs ensembles de caractéristiques de formation correspondant à un allèle d'antigène leucocytaire humain (HLA) identifié dans un échantillon de tumeur correspondant à un sujet d'un ensemble de sujets :
lesdites caractéristiques de formation comprenant, pour une région génomique de l'allèle HLA :
(1) une fréquence d'allèle B ajustée qui représente un rapport entre une première fréquence d'allèle B d'allèles hétérozygotes dans l'échantillon de tumeur qui correspond à la région génomique et une seconde fréquence d'allèle B d'allèles hétérozygotes dans la région génomique et associée à un ou plusieurs échantillons témoins ;
(2) un rapport entre une première couverture spécifique d'allèle de l'échantillon de tumeur qui correspond à la région génomique et une seconde couverture spécifique d'allèle du ou des échantillons témoins qui correspond à la région génomique ; et
(3) une indication indiquant si au moins une partie d'une région génomique flanquante entourant l'allèle HLA a été supprimée ;
la réception de données de séquence correspondant à un échantillon biologique d'un sujet particulier ;
la génération d'un résultat correspondant à une probabilité d'existence d'une perte d'hétérozygotie dans un allèle HLA identifié dans l'échantillon biologique du sujet particulier en traitant les données de séquence au moyen du modèle d'apprentissage automatique ; et
la délivrance en sortie du résultat ;
comprenant en outre la prédiction, sur la base du résultat, d'un ou plusieurs néoantigènes qui correspondent à l'allèle HLA identifié dans l'échantillon biologique du sujet particulier.

2. Procédé de la revendication 1, lesdites données de séquence étant des données de séquençage de l'exome entier.

3. Procédé de l'une quelconque des revendications 1 à 2, lesdites données de séquence étant des données de séquençage du génome entier.

4. Procédé de l'une quelconque des revendications 1 à 3, ledit modèle d'apprentissage automatique étant formé au moyen de l'ensemble de données de formation qui comprenait en outre une valeur de pureté tumorale et une valeur de ploïdie tumorale correspondant au sujet.

5. Procédé de l'une quelconque des revendications 1 à 4, ledit ensemble de données de formation étant généré au moyen d'une séquence de référence de l'allèle HLA correspondant au sujet.

6. Procédé de l'une quelconque des revendications 1 à 5, ledit modèle d'apprentissage automatique comprenant un ou plusieurs algorithmes d'amplification de gradient formés.

7. Procédé de l'une quelconque des revendications 1 à 6, comprenant en outre la prédiction, sur la base du résultat, d'une diminution de l'efficacité d'un traitement de blocage du point de contrôle immunitaire administré au sujet particulier.

8. Procédé de l'une quelconque des revendications 1 à 7, ledit échantillon biologique du sujet particulier comprenant une ou plusieurs cellules cancéreuses.

9. Procédé de l'une quelconque des revendications 1 à 8, ledit traitement des données de séquence au moyen du modèle d'apprentissage automatique comprenant la détermination de données spécifiques d'allèle pour un allèle HLA identifié à partir des données de séquence.

10. Procédé de la revendication 9, ledit allèle HLA étant identifié à partir des données de séquence par application d'un génotypage HLA aux données de séquence.

11. Système comprenant :
un ou plusieurs processeurs de données ; et
un support de stockage non transitoire lisible par ordinateur contenant des instructions qui, lorsqu'elles sont exécutées sur le ou les processeurs de données, amènent le ou les processeurs de données à réaliser tout ou partie d'un procédé selon l'une quelconque des revendications 1 à 10.

12. Produit-programme informatique incorporé de manière tangible dans un support de stockage non transitoire lisible par machine, comprenant des instructions configurées pour amener un ou plusieurs processeurs de données à réaliser tout ou partie d'un procédé selon l'une quelconque des revendications 1 à 10.
